# EUROPEAN PATENT SPECIFICATION

(11) **EP 1 384 477 B1**
(45) Date of publication and mention of the grant of the patent: **24.05.2006**
(21) Application number: 02714233.0
(22) Date of filing: 21.03.2002
(51) Int. Cl.: A61K 31/415, A61P 35/00, C07D 231/06

(54) **UTILIZATION OF PYRAZOLINE DERIVATIVES IN THE PREPARATION OF A MEDICAMENT FOR THE PREVENTION AND/OR TREATMENT OF PROLIFERATIVE CELL DISEASES**
VERWENDUNG VON PYRAZOLINDERIVATIVEN BEI DER HERSTELLUNG EINES MEDIKAMENTS ZUR PRÄVENTION UND/ODER BEHANDLUNG VON PROLIFERATIVEN ZELLERKRANKUNGEN
UTILISATION DE DERIVES DE PYRAZOLINES DANS L'ELABORATION D'UN MEDICAMENT POUR LA PREVENTION ET/OU LE TRAITEMENT DE MALADIES PROLIFERATIVES CELLULAIRES

(30) Priority: 06.04.2001 ES 200100818
(43) Date of publication of application: 28.01.2004
(60) Divisional application: 04030751.4
(73) Proprietor: LABORATORIOS DEL DR. ESTEVE, S.A., 08041 Barcelona (ES)
(72) Inventor: CUBERES-ALTISENT, Maria Rosa, E-08041 Barcelona (ES); BERROCAL-ROMERO, Juana Maria, E-08041 Barcelona (ES); CONTIJOCH-LLOBET, Maria Montserrat, E-08041 Barcelona (ES); FRIGOLA-CONSTANSA, Jordi, E-08041 Barcelona (ES)
(74) Representative: Carpintero Lopez, Francisco
(86) International application number: PCT/ES2002/000137
(87) International publication number: WO 2002/080909

(56) References cited:
- WO-A-00/76503
- US-A- 5 972 986

## Description

### Field of the Invention

The present invention relates to the use of pyrazoline derivatives with the general formula (I), as well as their physiologically acceptable salts, in the preparation of medicament useful in human and/or veterinary therapy for preventing or treating cell proliferation diseases, particularly for treatment of preneoplasic or neoplasic processes, tumoral angiogenesis, cachexia and processes related to tumoral necrosis factor (TNF) and in general any processes that can benefit from inhibiting the expression of the gene responsible for synthesis of cyclooxygenase-2 (COX-2), whether alone or in combination with other products, producing a synergy in the latter case.

### Background of the Invention

Our patent application number WO 99/62884 describes compounds with general formula (I) and their physiologically acceptable salts, 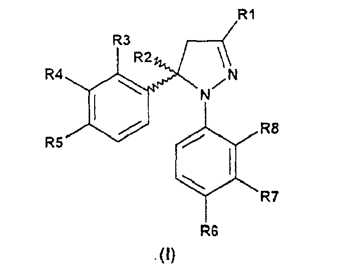 As inhibitors of the enzyme cyclooxygenase-2 (COX-2), with application in Medicine as anti-inflammatories.

The growing interest in the regulation of the gene responsible for synthesis of COX-2 derives from its application not only in its anti-inflammatory response but also in important pathological processes, such as cell proliferation and cancer, regulation of the immune response, degenerative diseases of the brain, etc., as made manifest in the growing related literature. A set of observations has led to considering COX-2 inhibitors as potential chemopreventive agents in colorectal cancer (CRC). The choice of COX-2 as a target is based on the frequency of its over-expression: up to 90% of carcinomas and 40% of adenomas of the colon show high amounts of mRNA and protein of COX-2 *(Eberhart et al. : Gastroenterology 1994 107 : 1183-1188; Du Bois et al. : Gastroenterology 1996 110: 1259-1262;* Prescott et *al., Cell 1996 87: 783-786)*. In addition, it seems clear that this over-expression contributes to the tumoral phenotype in CRC: a) overexpression of COX-2 has been related to inhibition of apoptosis *(Tsujii et al: Cell 1995 83: 493-501)*; b) inactivation of COX-2 in Apc (-) mice is associated with inhibited tumoral growth; c) two of the most common gene alterations in colorectal cancer, mutations in the Apc tumour suppression genes and mutations in ras oncogenes are related to the overexpression of COX-2 *(Boolbol et al: Cancer Res. 1996 56: 2556-2560; Sheng H. et al: J. Biol. Chem. 1998 273 (34): 2120-2127).*

Our patent application PCT/ES00/00245 describes a cell line comprised of a DNA construct consisting of all or part of a promoter sequence for the COX-2 gene and a token gene, operatively connected to each other so that said promoter sequence for the COX-2 gene directs the expression of said token gene in response to a suitable stimulus. The test method involves placing in contact said cell line with the compound to be tested and determining the existence of a signal indicating the expression of activity due to the token gene. This method is claimed as suitable for searching for selective inhibitors of the induction at a transcription level of OX-2 by suitable stimuli.

We have now found that the compounds of general formula (I), as well as their physiologically acceptable salts, are especially useful for preparing medicament of use in human and/or veterinary therapy for prevention or treatment of cell proliferation diseases, particularly for treatment of preneoplasic or neoplasic processes, angiogenesis, cachexia and processes related to tumoral necrosis factor (TNF) and, in general, processes which can benefit from inhibition of expression of the gene responsible for synthesising cylooxygenase2 (COX-2), whether alone or in combination with other products, in which case a synergy occurs.

### Detailed Description of the Invention

The present invention relates to the use of pyazoline derived of Δ²-pyrazoline, also known as 4,5-dihydro-1*H-*pyrazols, of general formula (I) 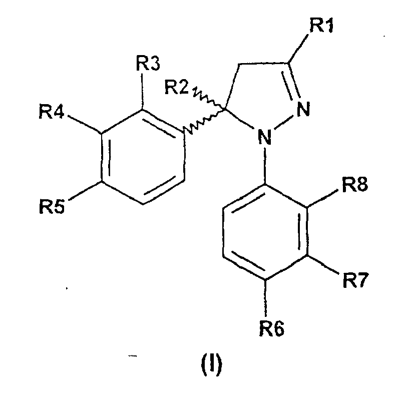 in the preparation of a medicament of use in human and/or veterinary therapy for prevention or treatment of cell proliferation diseases, particularly for the treatment of preneoplasic or neoplasic processes, angiogenesis, cachexia and processes related to tumoral necrosis factor (TNF) and, in general, processes which can benefit from inhibition of expression of the gene responsible for synthesising cylooxygenase2 (COX-2), whether alone or in combination with other products, in which case a synergy occurs.

In the formula (I)
R₁ represents an atom of hydrogen, a methyl, fluoromethyl, difluoromethyl, trifluoromethyl, carboxylic acid, alkyl carboxylate with less than 1 to 4 carbon atoms, carboxamide or cyano group,
R₂ represents an atom of hydrogen or a methyl group,
R₃, R₄, R₇ and R₈, like or different, represent an atom of hydrogen, chlorine, fluorine, a methyl, trifluoromethyl or methoxy group,
R₅ represents an atom of hydrogen, chlorine, fluorine, a methyl, trifluoromethyl, methoxy, trifluoromethoxy, methylsulphonyl, aminosulphonyl or acetylaminosulphonyl group
R₆ represents an atom of hydrogen, chlorine, fluorine, a methyl, trifluoromethyl, methoxy, trifluoromethoxy, methylsulphonyl, group,
with the condition that one of the substituents R₅ or R₆ is a methylsulphonyl, aminosulphonyl or acetylaminosulphonyl group, and
with the condition that when R₁ represents a methyl group
R₂ represents an atom of hydrogen or a methyl group,
R₃ and R₈, like or different, represent an atom of hydrogen, chlorine, fluorine, a methyl or trifluoromethyl group,
R₄ represents an atom of hydrogen, fluorine, a methyl, trifluoro-methyl or methoxy group,
R₅ represents an atom of fluorine, a trifluoromethyl, trifluoromethoxy, methylsulphonyl, aminosulphonyl or acetylaminosulphonyl group,
R₆ represents an atom of hydrogen, chlorine, fluorine, a methyl, trifluoromethyl, methoxy, trifluoromethoxy, methylsulphonyl, group,
with the condition that one of the substituents R₅ or R₆ is a methylsulphonyl, aminosulphonyl or acetylaminosulphonyl group, and
R₇ represents an atom of hydrogen, chlorine, fluorine, a methyl, trifluoromethyl or methoxy group.

The compounds with the general formula (I) have a stereogenic centre and thus can be prepared enantiomerically pure or as racemates. The racemates of compounds (I) can be resolved into their optical isomers by conventional methods, such as by chiral separation chromatography or by fractional crystallisation of their diastereoisomeric salts, which can be obtained by reacting the compounds (I) with enantiomerically pure acids or bases. Likewise, it they can also be obtained by enantioselective synthesis using enantiomerically pure chiral precursors.

The present invention also relates to the physiologically acceptable salts of the compounds with general formula (I), to the mineral and organic acid addition salts and to those formed with alkali metals.

The compounds with general formula (I), as well as their physiologically acceptable salts, inhibit the expression of the gene responsible for synthesis of cyclooxygenase-2 (COX-2) as can be shown with a cell system of stable transfectant JURKAT cells with the promoter of the gene COX-2 associated to the gene for luciferase, according to the method described in our patent application PCT/ES00/00245.

The compounds with general formula (I) can be used, administering a therapeutically effective dose, in mammals, including man, as agents for prevention or treatment of preneoplasic or neoplasic processes, partially or totally inhibiting the growth, propagation or metastasis of the neoplasia, as well as the partial or total destruction of neoplasic cells. For example, the compounds with general formula (I) can be used in neoplasias such as gastrointestinal cancer, liver cancer, bladder cancer, pancreatic cancer, lung cancer, prostate cancer, ovarian cancer, cervical cancer and breast cancer, or for prevention or treatment of adenomatose polyps including familiar polyposis.

The compounds with general formula (I) can be used, administering a therapeutically effective dose, in mammals, including man, as agents for prevention or treatment of diseases related to angiogenesis, such as tumoral growth and metastasis that rely on an angiogenic process, and in other disorders such as retinopathies and endometriosis.

The compounds with general formula (I) can be used, administering a therapeutically effective dose, in mammals, including man, as agents for prevention or treatment of cachexia and other disorders in which the tumoral necrosis factor-α (TNF-α) is involved.

The derivatives of general formula (I) can be prepared according to the methods described in our patent application WO 99/62884. Below is described, by way of example, the preparation of 1-(4-aminosulphonylphenyl)-5-(2,4-difluorophenyl)-4,5-dihydro-3-trifluoromethyl-1*H-*pyrazol (Example 3) and its enantiomers (Examples 79 and 80). Tables 1 and 2 show a family of compounds of particular interest which respond to the general formula (I), as well as their characterising physical-chemical properties.

### Example 3. - Preparation of 1-(4-aminosulphonylphenyl)-5-(2,4-difluorophenyl)-4,5-dihydro-3-trifluoromethyl-1H-pyrazol

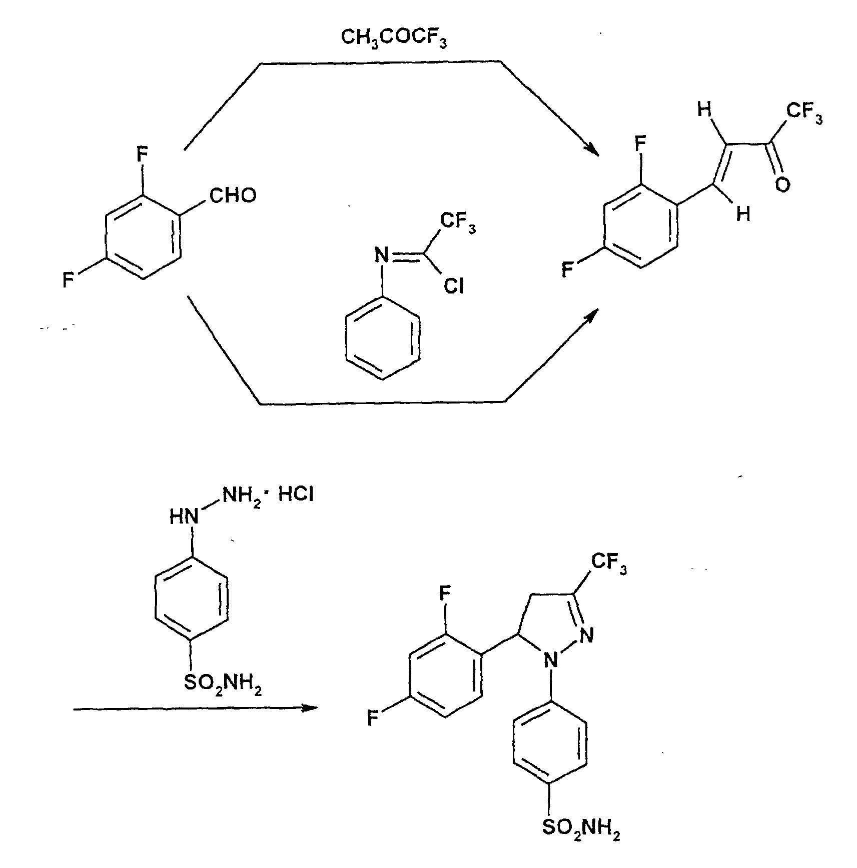

### Preparation, via acetymydoyl, of (E)-1,1,1-trifluoro-4-(2,4-difluorophenyl)-3-buten-2-one

In a flask with a dry inert atmosphere are introduced 260 ml of anhydrous THF, it is cooled to -78°C and 225 ml are added of LDA 2M solution in THF/n-heptane at a rate allowing to maintain the temperature under -65°C. After this diethylmethylphosphonate (34.25 g, 0.225 mol) is quickly added dropwise dissolved in 30 ml of THF and it is shaken for 30 minutes at -78°C. N-phenyltrifluoroacetymidoyl chloride is then added dropwise (46.7 g, 0.225 mol) dissolved in 40 ml of THF and it is left with shaking in the same conditions for 1 hour. A solution is added of 2,4-difluorobenzaldehyde (33.6 g, 0.236 mol) in 40 ml of THF, the cold bath is removed and the temperature is allowed to rise to ambient temperature. It is left shaking overnight in these conditions. The following morning 450 ml of HCl 2N are added and the shaking continued for 24 hours. The THF is removed in the rotovapor and the resulting aqueous solution is extracted with AcOEt (2x200 ml), washed with a solution of 5% NaHCO₃ and with a saturated NaCl solution, it is dried with sodium sulphate, filtered and the solvent evaporated with the rotovapor. In this way are obtained 54.6 g of a reddish liquid crude that solidifies. The crude is distilled at a pressure of 35 mbar and a fraction is collected of (E)-1,1,1-trifluoro-4-(2,4-difluorophenyl)-3-buten-2-one at 107-14°C (43 g, 81%). Melting point : 50-1°C
IR (KBr, cm⁻¹) : 1717, 1602, 1583, 1277, 1146, 1059, 706 ¹H-RMN (CDCl₃) : 6,9 (m, 2H), 7.05 (d, J=16 Hz, 1H), 7,6 (m, 1H), 8.0 (d, J=16Hz, 1H)
¹³C-RMN (CDCl₃): 105.1 (t,J=26Hz), 112,6(dd, J=4, 22Hz), 116.4 (q, J=291Hz), 118.2, 118.5, 131.5 (dd, J=4, 11Hz), 141.5, 162.5 (dd, J=13, 193Hz), 165,7 (dd, J=13, 190Hz), 180 (q, J=36Hz)

### Preparation via aldolic condensation of (E)-1,1,1-trifluoro-4-(2,4-difluorophenyl)-3-buten-2-one

In a flask are dissolved 2.4-difluorobenzaldehyde (250 g, 1.76 mol), glacial acetic acid (152.5 g, 2.54 mol) and piperidine (152.5 g, 1.79 mol) in 3 1 of THF. The solution is cooled to 5-10°C and CF₃COCH₃ (≅140 g, 1.2 mol) is bubbled through it. The cold bath is removed, the temperature is allowed to rise to ambient temperature and it is left with shaking at said temperature for 2 hours. CF₃COCH₃ (≅40 g, 0.36 mol) is added once again and left shaking for 2 hours. Another ≅40 g are added and shaken for another 2 hours, and so on until a total of about 415 g (3.7 mol) of CF₃COCH₃ have been added. Ammonium chloride 20% solution (600 ml) is added and the solvent is eliminated at a low pressure (50°C, 80 mbar). 300 ml of water are added and extracted with AcOEt. The organic phase is washed with water, H₂SO₄ 5%, water and dried over anhydrous sodium sulphate. It is filtered and evaporated. The resulting crude is distilled, to collect (30 mbar) a fraction (281.4 g, 68%) of (*E*)-1,1,1-trifluoro-4-(2,4-difluorophenyl)-3-buten-2-one with melting point 50-1°C. IR, ¹H-RMN and ¹³C-RMN identical to the product obtained via acetymydoyl.

### Preparation of 1-(4-aminosulphonylphenyl)-5-(2,4-difluorophenyl)-4,5-dihydro-3-trifluoromethyl-1H-pyrazol

A solution of 4-(aminosulphonyl)phenylhidrazine chlorhydrate (243.8 g, 1.09 mol) and (*E*)-1,1,1-trifluoro-4-(2,4-difluorophenyl)-3-buten-2-one (281.4 g) in 1600 ml of acetic acid is refluxed for 24 hours in a nitrogen atmosphere. It is cooled, poured slowly over water-ice (10-12 1) while vigorously shaken and filtered. It is washed with toluene (500 ml) and dried. 328 g of crude product are obtained (purity: 95.6%) which crystallises from dioxane. 216.7 g are obtained of 1-(4-aminosulphonylphenyl)-5-(2,4-difluorophenyl)-4,5-dihydro-3-trifluoromethyl-1*H*-pyrazol with a purity of 98.1%. The crystallisation liquor, once concentrated, provide a further 69.3 g with 97.5% purity. The union of these two fractions is recrystallised from isopropanol, yielding 267.8 g (61%) of ground product with a particle size < 100 µm, purity of 99.5% and melting point 161-2°C

| | | | | |
|---|---|---|---|---|
| Elementary analysis:% | C | %H | %N | %F |
| Calculated | 47.41 | 2.98 | 10.37 | 23.43 |
| Obtained | 47.42 | 2.77 | 10.35 | 23.57 |
| IR (KBr, cm⁻¹): | 3315, 1179, 3232, 1099, 1617, 1593, 1506, 1326, 1067 | | | |

¹H-RMN [300 MHz, CDCl₃, 25°C, δ (ppm)]: 3.0 (dd, J=6.3 and 11.4 Hz, 1H); 3,80 (dd, J=11.4 and 12,6 Hz, 1H); 4,79 (s wide, 2H); 5,70 (dd, J=6.3 and 12,6 Hz, 1H); 6,8-6,95 (m, 2H); 7.01-7.09 (m, 3H); 7,74 (d, J=8,7 Hz, 2H)
¹H RMN [300 MHz, DMSO-d₆, 25°C, δ (ppm)]: 3.13 (dd, J=18, 5 Hz, 1H); 3.89 (t, J=16 Hz, 1H); 5.96 (dd, J=13, 6 Hz, 1H); 7.03-7.16 (m, 5H); 7.33 (m, 2H); 7.64 (d, J=9 Hz, 2H)
¹³C-RMN [75 MHz, CDCl₃, 25°C, δ (ppm)]: 40.2, 57,9, 104,9 (t, J=25Hz), 112.4 (dd, J=4, 22Hz), 113.5, 120.4 (q, J=269Hz), 122.1 (d, J=17Hz), 128, 128.2, 133.5, 139.5 (q, J=38Hz), 145,8, 159,6 (dd, J=12, 245Hz), 163 (dd, J=12, 248Hz)
¹³C RMN [75 MHz, DMSO-d₆, 25°C, δ (ppm)]: 39.7, 58.8, 105.1 (t, J^{C-F}=26 Hz), 112.1 (dd, J^{C-F}=22, 3 Hz), 113.3, 120.9 (q, J^{C-F}=268 Hz), 123.1 (dd, J^{C-F}=14, 4 Hz), 127.4, 130.0 (dd, J^{C-F}=10, 5 Hz), 135.9, 139.0 (q, J^{C-F}=37 Hz), 144.6, 160.0 (dd, J^{C-F}=247, 13 Hz), 162.3 (dd, J^{C-F}=246, 13 Hz)
MS [EI, -70 eV, m/z (%)]: 405 (M⁺, 100), 386 (4), 341 (7), 292 (14), 156 (26), 139 (16)

### Preparation of (+)-1-(4-aminosulphonylphenyl)-5-(2,4-difluorophenyl)-4,5-dihydro-3-trifluoromethyl-1H-pyrazol (Example 79) and (-)-1-(4-aminosulphonylphenyl)-5-(2,4-difluorophenyl)-4,5-dihydro-3-trifluoromethyl-1H-pyrazol

### (Example 80)

The racemic mixture (±)-1-(4-aminosulphonylphenyl)-5-(2,4-difluorophenyl)-4,5-dihydro-3-trifluoromethyl-1*H-*pyrazol is resolved into its enantiomers by high resolution liquid chromatography using a CHIRALPAK AS column with particle size 10 µ and dimensions 25 x 2 cm (Daicel), mobile phase 0.1% of diethylamine in methanol and flow rate of 8 ml/min. With a retention time of 7.4 minutes (+)-1-(4-aminosulphonylphenyl)-5-(2,4-difluorophenyl)-4,5-dihydro-3-trifluoromethyl-1*H*-pyrazol is obtained as a white solid with m.p.: 173-4°C; enantiomeric purity 99.9 %; [α]_{D}=+183.9 (c=1 CH₃OH). With a retention time of 9.2 minutes (-)-1-(4-aminosulphonylphenyl)-5-(2,4-difluorophenyl)-4,5-dihydro-3-trifluoromethyl-1*H*-pyrazol is obtained as a white solid with m.p.: 173-4°C; enantiomeric purity >99,9%; [α]_{D}=-189.4 (c=1 CH₃OH).

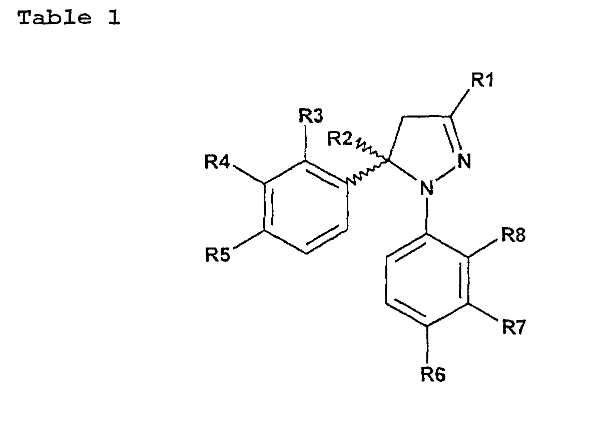

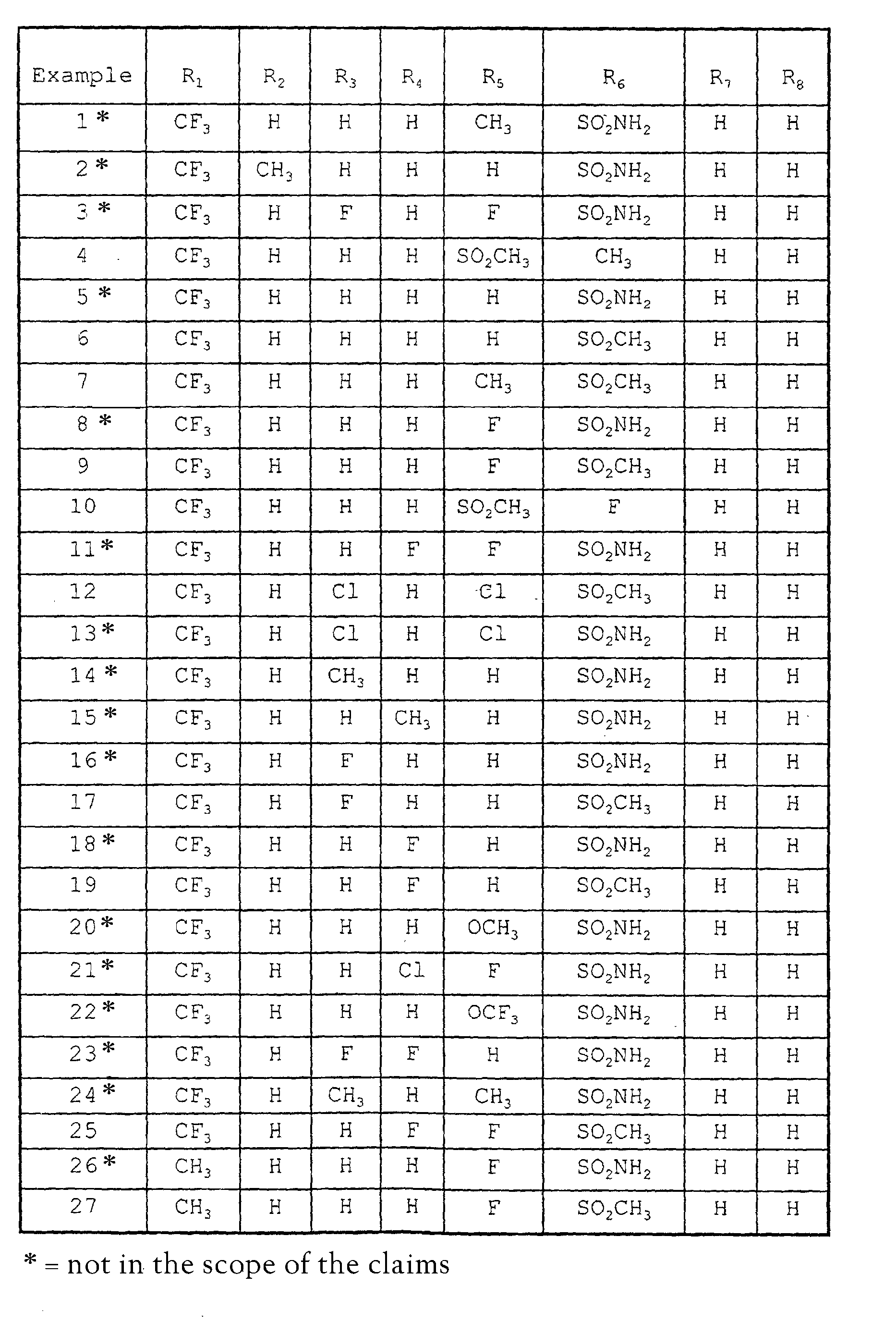

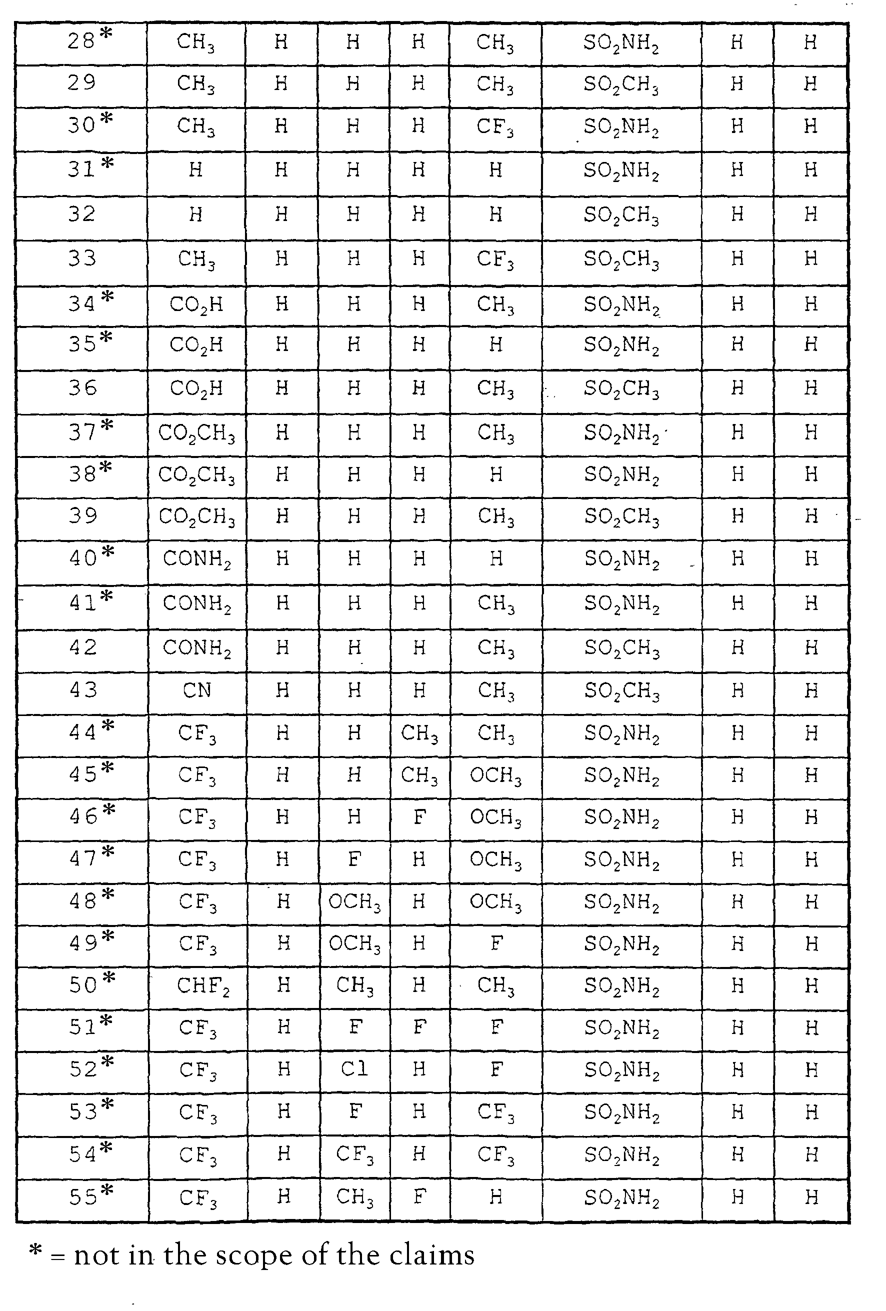

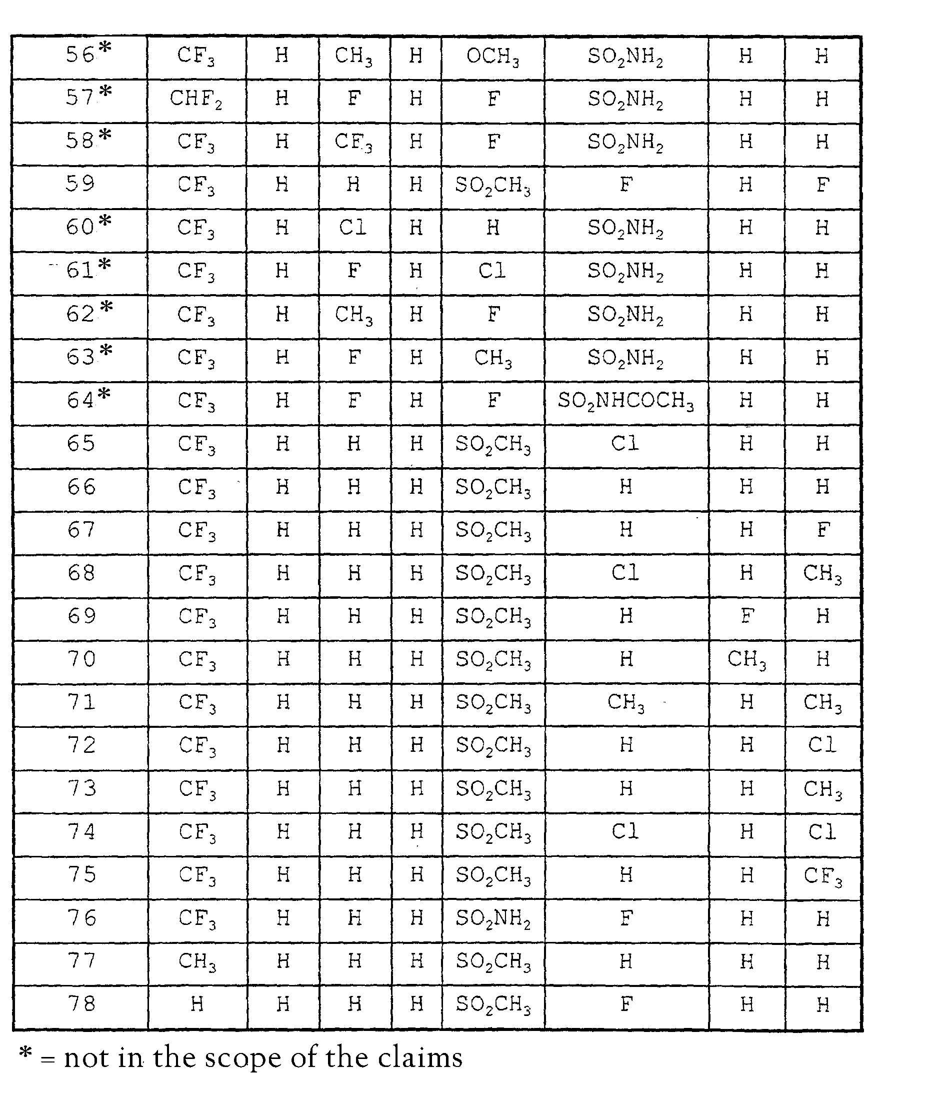

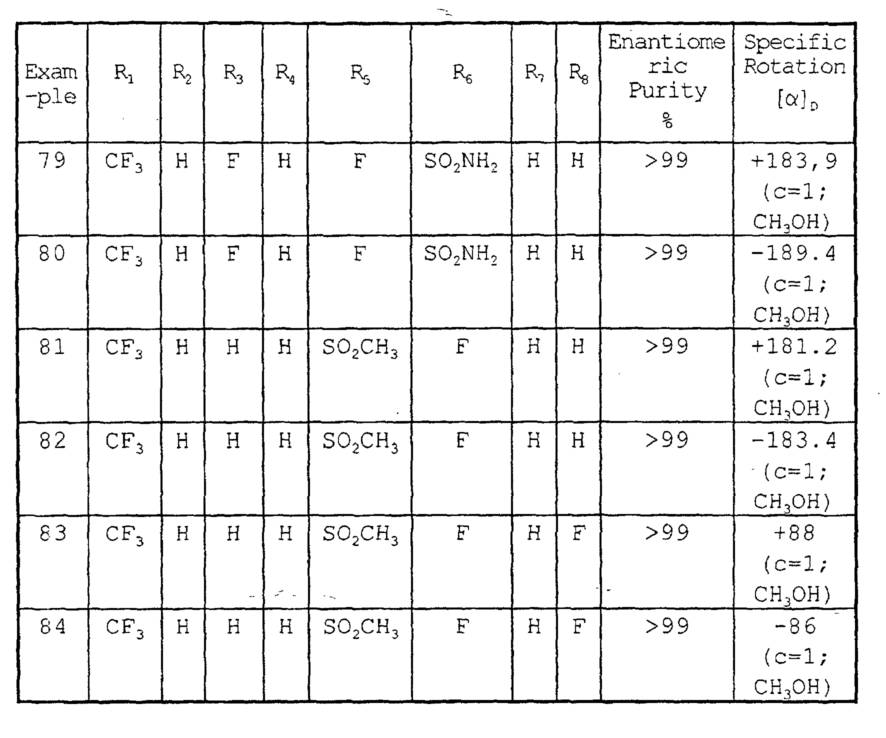

**Table 2**

| Ezamp le | m.p. °C | IR (KBr) cm⁻¹ | ¹H-RMN (CDCl₃) δ ppm |
|---|---|---|---|
| 1 | 140-3 | 3356, 3268, 1594, 1326, 1170, 1139, 1120, 1097 | 2.34(s,3H); 3 (dd, J=6.9, 14Hz, 1H); 3.7(dd, J=12.6, 14Hz.1H); 4.7(bs,2H); 5.4(dd, J=6.9, 12.6Hz.1H); 7.1(2d, J=8.1, 9.3Hz,4H); 7.2 (d, J=8. 1Hz, 2H) ; 7.7(d,J=9.3Hz,2H) |
| 2 | 60-6 | 3384, 3266, 1593, 1498, 1327, 1151, 1099, 703 | 1.6(s,3H); 2.8(m,1H); 3.1(m,1H); 4.5(bs, 2H); 7.2(m, 3H); 7.4-7.55(m, 4H); 7.7(d,2H) |
| 3 | 160-2 | 3315, 3232, 1617, 1593, 1506, 1326, 1179, 1099, 1067 | 3(dd,J=6.3, 11.4Hz,1H); 3.8(dd,J=11.4, 12.6Hz, 1H); 4.8(bs,2H); 5.7(dd,J=6.3, 12.6Hz,1H); 6.8-6.95(m,2H); 7-7.1(m,3H); 7.7(d,J=8.7Hz,2H) |
| 4 | 140-3 | 1516, 1310, 1148, 1131, 1060, 774 | 2.2(s,3H); 2.9(dd,J=7.8, 17.1Hz,1H); 3(s,3H); 3.7(dd,J=12.9, 17.1Hz,1H); 5.45(dd,J=7.8, 12.9Hz,1H); 6.8(d, J=8.4Hz,2H); 7(d, J=8.4Hz, 2H); 7.45(d,J=8.4Hz,2H); 7.9(d,J=8.4Hz,2H) |
| 5 | 156-7 | 3350,3269, 1596,1315 1188, 1142, 1101 | 3.04(dd, J=6.6, 18Hz,1H); 3.7(dd, J=12,9, 18 Hz,1H); 4.8(s,2H); 5.45(dd,J=6.6, 12, 9Hz, 1H); 7.0 (d, J=9Hz, 2H); 7.2(d,J=6.6Hz,2H); 7.3(m,3H); 7.7(d,J=9Hz,2H) |
| 6 | 137-40 | 1595, 1333, 1297, 1282, 1148, 771 | 3.0(s,3H); 3.06(dd,J=6.6, 18Hz,1H); 3.75(dd, J=12.8, 18.1H); 5.45(dd,J=6.6, 12.6Hz,1H); 7.05(d,J=9Hz,2H); 7.2(d,J=7.8Hz,2H); 7.4(m,3H); 7.7(d,J=9Hz,2H) |
| 7 | 115-19 | 1592, 1332, 1148, 1133, 825, 775 | 2.3(s,3H); 3.0(s,3H); 3.05(dd,J=6.6, 19Hz,1H) 3,7(dd,J=12.6, 19.1H); 5.4(dd,J=6.6, 12.6 Hz,1H); 7.1(2d,J=8.1, 8.7Hz,4H); 7.2(d,J=8.1 Hz,2H); 7.7(d,J=8.7Hz,2H) |
| 8 | 154-6 | 3337, 3254, 1594, 1510, 1324, 1158, 740 | 3.0(dd,J=6.6, 18Hz,1H); 3.7(dd,J=12.6, 18Hz, 1H); 4.8(s,2H); 5,4(dd,J=6.6, 12.6Hz,1H); 7.1(m,4H); 7.2(m,2H); 7.7(d,J=9Hz,2H) |
| 9 | 121-22 | 1592, 1509, 1148, 1120, 774 | 3.0(s,3H); 3.05(dd,J=6.6, 17,4Hz,1H); 3.7(dd, J=12.6, 17,4Hz,1H); 5.4(dd,J=6.6 y 12.6Hz, 1H); 7.0(m,4H); 7,2(m,2H); 7,7(d,J=9Hz,2H) |
| 10 | 103-5 | 1514, 1313, 1155, 1133, 1061, 827 | 2.9(dd,J=8,4, 17.4Hz,1H); 3(s,3H); 3.7(dd,J= 12.6, 17.4Hz,1H); 5.4(dd,J=8.4, 12.6Hz,1H); 6.9(m,4H); 7.45(d,J=8.4Hz,2H); 7.95(d,J=8.4 Hz,2H) |
| 11 | 153-5 | 3318, 3250, 1596, 1323, 1135, 1066 | 3(dd,J=6.9 y 18Hz, 1H); 3.7(dd,J=12.6.18Hz, 1H); 4.7(s ancho,2H); 5.4(dd,J=6,9, 12.6Hz, 1H); 7.0(m,4H); 7.2(m, 1H); 7.7(d,J=9Hz,2H) |
| 12 | 198-200 | 1596, 1320, 1303, 1138, 775 | 2.9-3.0(dd+s,4H); 3.85(dd, J=12.6, 18,3Hz, 1H) 5.8(dd, J=6.6, 12.6Hz,1H); 7.0(2d,J=9Hz, 3H); 7.2(d, J=9Hz,1H); 7.5(s, 1H); 7.8(d, J=9Hz,2H) |
| 13 | 143-5 | 3425, 3275, 1594, 1332, 1158, 1111, 825 | 2.95.(dd,J=6.3, 18.3Hz, 1H); 3.8(dd,J=12.3, 18,3Hz ,1H); 4.8(s,2H); 5.8(dd,J=6.3, 12.3Hz,1H); 7.0(2d,3H); 7.2(d,J=8.7Hz,1H); 7.5(s,1H); 7.7(d,J=8.1Hz,2H) |
| 14 | 124-6 | 3370, 3240, 1595, 1331, 1154, 1103 | (d₆-DMSO), 2.4(s,3H);2.9(dd,J=6.3, 18Hz,1H); 3.9(dd, J=13.2, 18Hz, 1H) ; 5.9(dd,J=6.3, 13.2Hz 1H); 6.8(s ancho, 1H); 7.0(d,J=9Hz,2H); 7.1 (m, 3H) ; 7.2(t,1H); 7.25(d,1H); 7.6(d, J=9Hz, 2H) |
| 15 | 125-8 | 3370, 3265, 1595, 1329, 1158, 1066 | (d₆-DMSO), 2.3(s,3H); 3(dd,J=6.3, 18.3Hz, 1H) 3.9(dd,J=12.6, 18.3Hz, 1H); 5.7(dd,J=6.3, 12.6Hz, 1H); 7-7.15(m, 5H); 7.25(t, 1H); 7.6(d, J=9Hz, 2H) |
| 16 | 166-8 | 3330, 3239, 1597, 1334, 1122, 769 | 3.05(dd,J=6.3, 17.7Hz,1H); 3.7(dd,J=12.6, 17.7Hz,1H); 5.7(dd,J=6.3, 12.6Hz,1H); 7-7.2(m,5H); 7.3(m,1H); 7.7(d,J=9Hz,2H) |
| 17 | 117-121 | 1594, 1304, 1150, 1119, 776 | 3(s,3H);3.05(dd,J=6.6, 17.1Hz,1H); 3.8(dd,J= 12.9, 17.1Hz,1H); 5.75(dd,J=6.6, 12, 9Hz, 1H) ; 7-7.2(m,5H); 7.3(m,1H); 7.75(d,J=9Hz,2H) |
| 18 | 132-3 | 3323, 3249, 1596, 1323, 1179, 1131, 741 | 3(dd,J=7.2, 16.8Hz,1H); 3.75(dd,J=12.9, 16.8Hz,1H); 4.8(s ancho,2H); 5.4(dd,J=7.2, 12.9Hz,1H); 6.9(d,J=9Hz,1H); 7.05(m,4H); 7.4(m,1H); 7.7(d,J=9Hz,2H) |
| 19 | 149-151 | 1593, 1296, 1144, 965, 789 | 3(s+dd,4H); 3.75(dd,J=12.6, 13.8Hz,1H); 5.4(dd,J=6.9, 12.6Hz,1H); 6.9-7.1(m,5H); 7.4(m,1H); 7.7(d,J=9Hz,2H) |
| 20 | 125-8 | 3336, 3254, 1593, 1329, 1156, 1112, 834 | 3(dd,J=6.6, 18Hz,1H); 3.7(s+dd,4H); 4.75(s ancho,2H); 5.4(dd,J=6.6, 12.9Hz,1H); 6.9(d,J=8.4Hz,2H); 7.05(d,J=8.4Hz,2H); 7.1(d,J=8.4Hz,2H); 7.7(d,J=8.4Hz,2H) |
| 21 | 171-3 | 3376, 3239, 1593, 1500, 1328, 1153 | 3(dd,J=6.9, 18.3Hz,1H); 3.75(dd,J=12.6, 18.3Hz,1H); 4.7(s ancho,2H); 5.4(dd,J=6,9, 12.6Hz, 1H); 7-7.2 (m, 4H); 7. 3 (m,1H); 7.7(d,J=8.7Hz,2H) |
| 22 | 134-7 | 3386, 3265, 1595, 1259, 1159 | (d₆-DMSO): 3(dd,J=6, 18.3Hz,1H); 3.9(dd,J=12.9, 18.3Hz,1H); 5.9(dd,J=6, 12.9Hz,1H); 7.05(d,J=8.7Hz,2H); 7.1(s ancho,2H); 7.4(s,4H); 7.6(d,J=8.7Hz,2H) |
| 23 | 152-4 | 3334, 3237, 1595, 1331, 1128, 831 | 3.05(dd,J=6.6, 18.6Hz,1H); 3.8(dd,J=12.9, 18.6Hz,1H); 4.7(s ancho, 2H); 5.7(dd,J=6,6, 12.9Hz,1H); 6.8(m,1H); 7-7.2 (m, 4H); 7.7(d,J=7.8Hz, 2H) |
| 24 | 158-160 | 3361, 3270, 1593, 1325, 1168, 1140, 821 | 2.3(s,3H); 2.4(s,3H); 2.9(dd,J=6.9, 17.7Hz,1H); 3.8(dd, J=12.9, 17.7Hz,1H); 4.7(b s, 2H); 5.6(dd,J=6.9, 12.9Hz,1H); 6.8-7.0(m, 4H); 7.1(s,1H); 7.7(d,J=8.4Hz,2H) |
| 25 | 132-5 | 1595, 1325, 1281, 1135, 774 | 3(s+dd, 4H); 3.8(dd,J=6.6, 18Hz,1H); 5.45(dd,J=12.6, 18Hz,1H); 6.9-7.05(m, 4H); 7.2(m,1H); 7.75(d,J=9Hz,2H) |
| 26 | 206-8 | 3329, 3215, 1593, 1509, 1333, 1155, 817 | (d₆-DMSO): 2(s,3H); 2.65(dd,J=5.6, 20Hz,1H); 3.55(dd,J=12.6, 20Hz,1H); 5.35(dd,J=5.6, 12.6Hz,1H); 6,8(d,J=8.4Hz,2H); 6.95(s,2H); 7.1-7.25(m,4H); 7.5(d,J=8.4Hz,2H) |
| 27 | 120-3 | 1590, 1508, 1293, 1141 | 2.1(s,3H); 2.7(dd,J=6, 18.3Hz,1H); 2.95(s,3H); 3.5(dd,J=12, 18.3Hz,1H); 5,1(dd,J=6, 12Hz,1H); 6.9(d,J=9Hz,2H); 7(m,2H); 7.2(m,2H); 7.6(d,J=9Hz,2H) |
| 28 | 195-7 | 3300, 3210, 1594, 1509, 1330, 1157 | (d₄ -CH₃OH): 2(s,3H); 2.2(s,3H); 2.6(dd,J=5.4, 17.7Hz,1H); 3.5(dd,J=11.7, 17.7Hz,1H); 5.3(dd,J=5.4, 11.7Hz,1H); 6.8(d,J=8.7Hz,2H); 6.9(s,2H); 7.1(m,4H); 7.5(d,J=8.7Hz,2H) |
| 29 | 113-7 | 1592, 1509, 1298, 1142, 771 | 2.1(s,3H); 2.3(s,3H); 2.7(dd,J=6.3, 20Hz,1H); 2.95(s,3H); 3.5(dd,J=13, 20Hz,1H); 5.1(dd,J=6.3, 13Hz,1H); 6.9(d,J=9Hz,2H); 7.1(m,4H); 7.6(d,J=9Hz,2H) |
| 30 | 190-4 | 3344, 3263, 1596, 1329, 1155, 616 | (d₄-CH₃OH): 2.9-(dd,J=6, 18.3Hz,1H); 3.7(dd, J=12, 18.3Hz,1H); 5.3(dd,J=6, 12Hz,1H); 7.1(m,3H); 7.4(m,5H); 7.7(d,J=8.7Hz,2H) |
| 31 | 206-8 | 1595, 1290, 1144, 774 | 2.9(s+dd,4H); 3.6(dd,J=12.3, 18.3Hz,1H); 5.1(dd,J=6.3, 12.3Hz,1H); 6.9(s,1H); 7(d,J=9Hz,2H); 7.3(m,5H); 7.7(d,J=9Hz,2H) |
| 32 | 197-202 | 3320, 3250, 1594, 1325, 1165 | (d₆-DMSO): 2 (s, 3H) ; 2.7(dd,J=5.4, 18Hz,1H); 3.6(dd,J=12, 18Hz,1H); 5.5(dd,J=5.4, 12Hz, 1H); 6.85(d,J=8.1Hz,2H); 7(s,2H); 7.4(d,J=8,1 Hz,2H); 7.5(d,J=8.1Hz,2H); 7.7(d,J=8.1Hz,2H) |
| 33 | 136-8 | 1595, 1512, 1325, 1141, 771 | 2.1(s,3H); 2.7(dd,J=6.3, 19Hz,1H); 3(s,3H); 3.5(dd,J=12.6, 19Hz,1H); 5.2(dd,J=6.3, 12.6Hz,1H); 6.9(d,J=8.4Hz,2H); 7.35(d,J=8.4Hz,2H); 7.6(2d,4H) |
| 34 | 172-6 | 3304, 3237, 1706, 1326, 1138, | (d₄-CH₃OH) : 2.35(s,3H); 3.05(dd,J=6.6, 18.6Hz,1H); 3.8(dd,J=12.6, 18.6Hz,1H); 5.5(dd,J=6.6, 12.6Hz,1H); 7.2(m, 6H), 7.7(d,J=9Hz,2H) |
| 35 | 157-164 | 3247, 1700, 1595, 1333, 1150, 1098 | (d₄-CH₃OH): 3.1(dd,J=6, 18.3Hz,1H); 3.9(dd,J=12.6, 18.3Hz,1H); 5.7(dd,J=6, 12.6Hz,1H); 7.2-7.5(m,7H); 7.7(d,J=8.7Hz,2H) |
| 36 | 202-5 | 1730, 1582, 1275, 1206, 1134, 1087 | (d₆-DMSO): 2.2(s,3H); 2.8(dd,J=6.3, 18Hz,1H); 3.05(s,3H); 3.8(dd,J=12.6, 18Hz,1H); 5.7(dd,J=6.3, 12.6Hz,1H); 7,2(m, 6H); 7.7(d,J=9Hz,2H); 13.2(b s, 1H) |
| 37 | 192-7 | 3306, 3231, 1706, 1324, 1158 | 2.2(s,3H); 3(dd,J=6.3,18Hz,1H); 3.2(b s, 2H); 3.65(dd,J=12.6, 18Hz,1H); 3.8(s,3H); 5.4(dd,J=6,3, 12.6Hz,1H); 7-7.1(m,6H); 7.6(d,J=8.7Hz,2H) |
| 38 | 84-90 | 3308, 3224, 1700, 1317, 1147, 1094 | (d₄-CH₃OH): 3.1(dd,J=6, 18.3Hz,1H); 3.9(s+dd, 4H); 5.7(dd,J=6, 12.9Hz,1H); 7.2-7.4(m, 7H); 7.75(d,J=8.7Hz,2H) |
| 39 | 155-160 | 1741, 1561, 1260, 1226, 1135, 1089 | 2.3(s,3H); 3(s,3H); 3.1(dd,J=6, 18.3Hz,1H); 3.75(dd,J=12.6, 18,3Hz,1H); 5.4(dd,J=6, 12.6Hz,1H); 7-7,25(m,6H); 7.7(d,J=8.7Hz,2H) |
| 40 | 200-5 | 3431, 3285, 1647, 1592, 1328, 1142 | (d₄-CH₃OH): 3.1(dd,J=6, 18,3Hz,1H); 3.9 (dd,J=12.9, 18.3Hz,1H); 5.7(dd,J=6, 12.9Hz, 1H); 7.2-7.5(m, 7H); 7.75(d,J=8.7Hz,2H) |
| 41 | 210-5 | 3950, 3337, 1656, 1596, 1345, 1141 | (d₄-CH₃OH): 2.4(s,3H); 3.05(dd,J=6, 17.7Hz,1 H); 3.8(dd,J=12.9, 17.7Hz,1H); 5.6(dd,J=6, 12.9 Hz,1H);7.2-7.3(m,6H) 7.75(d,J=8.7Hz,2H) |
| 42 | 128-132 | 3440, 3200, 1680, 1590, 1135 | 2.3(s,3H); 3(s,3H); 3.1(dd,J=6.3,18.6Hz,1H); 3.8(dd,J=12.6, 18.6Hz,1H); 5.4(dd,J=6.3, 12.6Hz,1H); 5.6(s ancho,1H); 6.7(s ancho,1H); 7-7.2(m, 6H); 7.7(d,J=8.7Hz,2H) |
| 43 | 162-4 | 2220, 1593, 1500, 1389, 1296, 1143 | 2.3(s,3H); 3-3.1(s+dd,4H); 3.75(dd,J=12.6, 18Hz,1H); 5.5(dd,J=6.3, 12.6Hz,1H); 7-7.2(m,6H); 7.7(d,J=8.7Hz,2H) |
| 44 | 152-5 | 3316, 3240, 1594, 1323, 1178, 1121, 1065, 549 | 2.2(s,6H); 3(dd,J=6.3, 18.3Hz,1H); 3.7(dd, J=12.6, 18.3Hz,1H); 4,7(s ancho,2H); 5.4(dd, J=6.3, 12.6Hz,1H); 6.95(s+d,J=7.8Hz,2H); 7.1(2d,J=7,8, 8.7Hz,3H); 7.7(d,J=8.7Hz,2H) |
| 45 | 170-5 | 3360, 3267, 1595, 1507, 1329, 1255, 1159, 619 | 2.2(s,3H); 3(dd,J=7.2, 18Hz,1H); 3.6-3.8(s+dd,4H); 4.6(b s, 2H); 5.35(dd,J=7,2, 12.9Hz,1H); 6.75(d,J=7.8Hz,1H); 7(s+d,2H); 7.1(d,J=8.7Hz,2H); 7.7(d,J=8.7Hz,2H) |
| 46 | 108-14 | 3383, 2270, 1595, 1519, 1329, 1277, 1160, 1066 | 3(dd,J=6,6 18.3Hz,1H); 3,75(dd,J=12.3, 18.3 Hz,1H); 3.9(s,3H); 5.4(dd,J=6.6, 12.3Hz,1H); 6.95(m,3H); 7.05(d,J=8.7Hz,2H); 7.7(d,J=8.7Hz,2H) |
| 47 | 157-9 | 3357, 3267, 1630 1595, 1508, 1330, 1264, 1158, 1066 | 3.05(dd,J=6.3, 18Hz,1H); 3.7-3.8(s+dd, 4H); 4.8(s ancho,2H); 5.7(dd,J=6.3, 12.9Hz,1H); 6.6-6.7(m,2H); 6.95(t,J=8.7Hz,1H); 7.05(d,J=9Hz,2H); 7.7(d,J=9Hz,2H) |
| 48 | 121-6 | 3376, 3268, 1593, 1507, 1329, 1160 | 2.9(dd,J=6, 18Hz,1H); 3.65(dd,J=12,6, 18Hz,1H); 3.75(s,3H); 3.85(s,3H); 4.9(s,2H); 5.65(dd,J=6, 12.6Hz,1H); 6.35(d,J=8,7Hz,1H); 6.5(s,1H); 6.9(d,J=8.7Hz,1H); 7 (d, J=8.7Hz,2H); 7.7(d,J=8.7Hz,2H) |
| 49 | 179-82 | 3317, 3231, 1593, 1507, 1326, 1178 | (d₆-DMSO): 2.95(dd, J=5.4, 18Hz, 1H); 3.7-3.8(m,4H); 5.8(dd,J=5.4, 12.6Hz,1H); 6.7(dd,J=8.1, 10.5Hz, 1H); 6.9-7,1 (m, 6H) ; 7.6(d,J=8.7Hz,2H) |
| 50 | 181-3 | 3348, 3268, 1593, 1321, 1165 | 2.25(s,3H); 2.35(s,3H); 2.85(dd,J=6.9, 18Hz,1H); 3.7(dd,J=12.6, 18Hz,1H); 5.45(dd,J=6.9, 12.6Hz,1H); 6.5(t,J=54Hz,1H); 6.8-6.9(m,4H); 7(s,1H); 7.65(d,J=9Hz,2H) |
| 51 | 159-61 | 3382, 3285, 1595, 1514, 1328, 1161 | 3(dd,J=6.3, 17.7Hz,1H); 3.8(dd,J=12.6, 17.7Hz,1H); 4.7(s,2H); 5.7(dd,J=6.3, 12.6Hz,1H); 6.8(m,1H); 6.9(m,1H); 7(d,J=9Hz,2H); 7.75(d,J=9Hz,2H) |
| 52 | 167-9 | 3318, 3239, 1593, 1503, 1492, 1321, 1068 | (d₆-DMSO): 3(dd,J=0.3, 18.3Hz,1H); 3.95(dd,12.9, 18.3Hz,1H); 5.95(dd,J=6.3, 12.9Hz,1H); 7(d,J=8.7Hz,2H); 7.1-7.2(m,4H); 7.55(d,J=8.4Hz,1H); 7.65(d,J=8.7Hz,2H) |
| 53 | 170-3 | 3425, 3284, 1595, 1330, 1138 | (d₆-DMSO) : 3.2(dd,J=5.7, 18Hz,1H); 3,9(dd,J=12.9, 18Hz,1H); 6(dd,J=5.7, 12.9Hz,1H); 7.1(m,4H); 7.4-7.7(m,4H); 7,8(d,J=10.8Hz,1H) |
| 54 | 212-4 | 3376, 3277, 1597, 1332, 1274, 1132 | 2.8(dd,J=6.3, 18.5Hz,1H); 3.7(dd,J=13, 18.5Hz,1H); 5.75(dd,J=6.3, 13Hz,1H); 6.1(s,2H); 6.8(d,J=8.5Hz,2H); 7.2(d,J=8.3Hz,1H); 7.6(d,J=8.5Hz,2H); 7.65(d,J=8.3Hz,1H); 7.9(s,1H) |
| 55 | 193-5 | 3353, 3270, 1593, 1509, 1321, 1141 | (d₆-DMSO): 2.3(s,3H); 2.9(dd,J=6.1, 12.2Hz,1H); 3.95(dd,J=12.2, 12.9Hz,1H); 5.95(dd, J=6.1, 12.9Hz,1H); 6.65(s ancho,1H); 7(d,J=8,8Hz,2H); 7.1-7.2(m,4H); 7.65(d,J=8.8Hz,2H) |
| 56 | 148-50 | 3384, 3266, 1593, 1324, 1252, 1166 | 2.35(s,3H); 2.9(dd,J=5.6, 18Hz,1H); 3.7-3.8(m,4H); 4.9(banda ancha,2H); 5.5(dd,J=5.6, 12.6Hz,1H); 6.6(ddJ=2.2, 8.5Hz,1H); 6. 8 (s, 1H) ; 6.85-6.95(2d,3H); 7.7(d,J=9Hz,2H) |
| 57 | 157-60 | 3384, 3346, 3277, 3255, 1596, 1503, 1341, 1158 | 3(dd,J=6.1, 17.8Hz,1H); 3.7(dd,J=12.4, 17.8Hz,1H); 4.75(s,2H); 5.6(dd,J=6.1, 12.4Hz,1H); 6.5(t,J=54Hz,1H); 6.8-7(m,5H); 7.7(d,J=8.8Hz,2H) |
| 58 | 174-7 | 3384, 3261, 1596, 1329, 1117 | 2.95(dd,J=5.6, 17.3Hz,1H); 3,75(dd,J=12,4, 17.3Hz,1H); 4.7(s ancho, 2H); 5.8(dd,J=5.6, 12.4Hz,1H); 6.95(d,J=8.3Hz,2H); 7.2(m,2H); 7.5(d,J=7.5Hz,1H); 7.75(d,J=8.3Hz,2H) |
| 59 | 105-6 | 1596, 1510, 1314, 1264, 1150, 845 | 3(s+dd, 4H); 3.6 (dd, J=12.2, 17.6Hz, 1H); 5.6 (dd, J=6,2, 12.2Hz, 1H); 6.65 (t, J=9Hz, 1H); 6,75 (t, J=8Hz, 1H); 7.35 (m, 3H); 7.8 (d, J=8.3Hz, 2H) |
| 60 | 157-9 | 3354, 3268, 1594, 1325, 1122, 753 | 2.95(dd,J=6.6, 18.5Hz,1H); 3.85(dd,J=12,7, 18.5Hz,1H); 4.8(s,2H); 5.8(dd,J=6.6, 12.7Hz,1H); 6,9-7(m,3H); 7.1-7.3(m,2H); 7.45(d,J=7.8Hz,1H); 7.7(d,J=8.6Hz,2H) |
| 61 | 180-5 | 3407, 3295, 1593, 1334, 1161 | (d₄-CH₃OH): 3.2(dd,J=6.3, 18.1Hz,1H); 3.95(dd(J=12.9, 18.1Hz,1H); 6(dd,J=6.3, 12.9Hz,1H); 7.2(d,J=8.8Hz,2H); 7.3(m,2H); 7.4(d,J=10.3Hz,1H); 7.8(d,J=8.8Hz,2H) |
| 62 | 154-60 | 3406, 3262, 1593, 1330, 1155 | 2.4(s,3H); 2.9(dd,J=6.6, 17.8Hz,1H); 3.75(ddJ=12, 7, 17.8Hz,1H); 4.8(s,2H); 5.5(dd,J=6.6, 12.7Hz,1H); 6.8-7(m,5H); 7.7(d,J=8.8Hz,2H) |
| 63 | 166-7 | 3430, 3298, 1593, 1508, 1334, 1161, 1123 | 2.3(s,3H); 3 (dd, J=6. 3, 18.3Hz,1H); 3.75(dd,J=12, 7, 18.3Hz,1H); 4.65(s,2H); 5.7(dd,J=6.3, 12.7Hz,1H); 6.85-7(m,3H); 7.05(d,J=8.8Hz,2H); 7.7(d,J=8.8Hz,2H) |
| 64 | 172-4 | 3302, 1722, 1593, 1506, 1337, 1165 | 2 (s, 3H) ; 3(dd,J=6.6, 18Hz,1H); 3.8(dd,J=12.9, 18Hz,1H); 5.7(dd,J=6.6, 12.9Hz,1H); 6.8-6.95(m,2H); 7-7.1(m,3H); 7.85(d,J=8.7Hz,2H); 8.1(s,1H) |
| 65 | 117-21 | 1594, 1492, 1310, 1257, 1154, 1063 | 2.95(dd,J=7.3, 17.8Hz,1H); 3(s,3H); 3.7(dd,J= 12.7, 17.8Hz,1H); 5.45(dd,J=7.3, 12.7Hz,1H); 6.8(d,J=8.8Hz,2H); 7.1(d,J=8.8Hz,2H); 7.4(d,J=8.3Hz,2H); 7.9(d,J=8.3Hz,2H) |
| 66 | 114-5 | 1598, 1503, 1275, 1156, 1079, 749 | 2.95(dd,J=7.6, 17.8Hz,1H); 3 (s, 3H) ; 3.7(dd,J= 12.7, 17.8Hz,1H); 5.45(dd,J=7.6, 12.7Hz,1H); 6.9(m,3H); 7.15(t,J=7.8Hz,2H); 7.4(d,J=8,1Hz,2H); 7.9(d,J=8.1Hz,2H) |
| 67 | 98-9 | 1606, 1503, 1317, 1148, 1123, 762 | 3(s+dd,4H); 3.65(dd,J= 13.1, 17.1Hz,1H); 5.8(dd,J=7.6, 13,1Hz,1H); 6.9(m,2H); 7(t,J=8.1Hz,1H); 7.3(d,J=8.1Hz,2H); 7.45(t,J=8.3Hz,1H); 7.8(d,J=8.1Hz,2H) |
| 68 | 104-8 | 1617, 1496, 1310, 1253, 1154, 1113, 809 | 2,3(s,3H); 3(m,4H); 3.5(dd,J= 11.7, 17.1Hz, 1H); 5.45(t,J=11.7Hz,1H); 6.75(d,J=8.5Hz,1H); 7(d,J=8.5Hz,1H); 7.1(s,1H); 7.45(d,J=8Hz,2H); 7,9(d,J=8Hz,2H) |
| 69 | 116-7 | 1616, 1587, 1498, 1310, 1155, 828 | 2.9(dd,J=7.5, 16.8Hz,1H); 3(s,3H); 3.7(dd,J= 12.7, 16.8Hz,1H); 5.4(dd,J=7.5, 12.7Hz,1H); 6.6(m,2H); 6.7 (d, J=11Hz, 1H) ; 7,1(dd, J=7.6, 14.9Hz, 1H); 7.4(d, J=8Hz, 2H); 7.9(d, J=8Hz, 2H) |
| 70 | 114-6 | 1597, 1315, 1149, 1072, 959, 789 | 2.25(s,3H); 2.9(dd,J=7.6, 17.8Hz, 1H); 3(s,3H); 3.7(dd,J= 12.9, 17.8Hz, 1H); 5.45(dd,J=7.6, 12.9Hz, 1H); 6.6(d, J=7.8Hz, 1H); 6.7(d, J=7.8 Hz, 1H); 6.9(s,1H); 7(t,J=7.8Hz,1H); 7.45(d,J=8Hz,2H); 7.9(d,J=8Hz,2H) |
| 71 | 132-3 | 1601, 1509, 1314, 1154, 1113, 809 | 2.2(s,3H); 2.3(s,3H); 3(m,4H); 3.5(dd,J= 11.7, 16.6Hz, 1H); 5.4(t,J=11.7Hz,1H); 6.8(m,2H); 6.9(s,1H); 7.5(d,J=8Hz,2H); 7.85(d,J=BHz,2H) |
| 72 | | 1617, 1589, 1483, 1313, 1149, 759 (film) | 2.95(s,3H); 3.15(dd,J=6.5, 17.8Hz,1H); 3.65(dd,J= 12.7, 17.8Hz,1H); 5.95(dd,J=6.5, 12.7Hz,1H); 6.95(d,J=7.8Hz,1H); 7.1(t,J=7.3Hz,1H); 7.2(m,2H); 7.35(d,J=8.3Hz,2H); 7.8(d,J=8.3Hz,2H) |
| 73 | | 1598, 1496, 1406, 1312, 1151, 757 (film) | 2.3(s,3H); 3(s+dd,4H); 3.5(dd,J= 11.7, 17.8Hz,1H); 5.5(t,J=11.7Hz,1H); 6.85 (d,J=7.8Hz,1H); 7(m,2H); 7.1(d,J=6.1Hz,1H); 7.5(d,J=8.3Hz,2H); 7.85(d,J=8.3Hz,2H) |
| 74 | 103-6 | 1625, 1483, 1312, 1150, 1130, 819 | 3(s,3H); 3.15(dd,J=5.9, 17.8Hz, 1H); 3.7(dd,J= 11.7, 17.8Hz, 1H); 5. 95 (dd, J=5. 9, 11.7Hz,1H); 7.05 (d,J=8.8Hz,1H); 7.2(m,2H); 7.3(d,J=8.1Hz,2H); 7.8(d,J=8.1Hz,2H) |
| 75 | | 1603, 1318, 1148, 1060, 955, 760 (film) | 3(s,3H); 3.1(dd,J=8.8, 16.4Hz, 1H); 3.6(dd,J= 12.7, 16.4Hz, 1H); 5.6(dd,J=8.8, 12.7Hz, 1H); 7(d, J=7.8Hz, 1H); 7.15(t,J=8.1Hz,1H); 7.3(t, J=8.1Hz, 1H); 7.4(d,J=8.3Hz,2H); 7.6(d,J=7.8Hz,1H); 7.8(d,J=8.3Hz,2H) |
| 76 | 138-40 | 3340, 3249, 1508, 1332, 1165, 1121, 832 | (d₆-DMSO): 3(dd, J=7.6, 16.1Hz,1H); 3.8(dd, J=12.9, 16.1Hz,1H); 5.8(dd, J=7.6,12.9Hz,1H); 6.9(m,2H); 7.1(t, J=8.9Hz,2H); 7.35(s ancho, 2H); 7.4(d,J=8.3Hz,2H); 7.8(d,J=8.3Hz,2H) |
| 77 | 132-5 | 1598, 1508, 1303, 1149, 744 | 2.1(s,3H); 2.7(dd,J=7.8, 17.3Hz, 1H); 3(s,3H); 3.5(dd,J=12.2, 17.3Hz,1H); 5,1(dd,J=7.8, 12.2Hz, 1H); 6.8(t, J=7.1Hz,1H); 6.9(d, J=8Hz, 2H); 7.1(t, J=8Hz,2H); 7.5(d,J=8Hz,2H); 7.9(d, J=8Hz,2H) |
| 78 | 155-60 | 1510, 1290, 1140, 800, 540 | 2.8(dd,J=8.3, 17,8Hz,1H); 3(s,3H); 3.5(dd,J=12.2, 17.8Hz, 1H); 5(dd,J=8.3, 12.2Hz,1H); 6.8-6.95(m,5H); 7.5(d, J=8.3Hz,2H)); 7.9(d,J=8.3Hz,2H) |
| 79 | 173-4 | 3330, 3250, 1617, 1593, 1506, 1329, 1121, 1099, 855 | 3(dd,J=6.3, 11.4Hz,1H); 3.8(dd,J=11.4, 12.6Hz, 1H); 4.8(s ancho,2H); 5.7(dd,J=6.3, 12.6Hz,1H); 6.8-6.95(m,2H); 7-7.1(m,3H); 7.7(d,J=8.7Hz,2H) |
| 80 | 173-4 | 3330, 3250, 1617, 1593, 1506, 1329, 1121, 1099, 855 | 3(dd,J=6.3, 11.4Hz,1H); 3.8(dd,J=11.4, 12.6Hz, 1H); 4.8(s ancho,2H); 5.7(dd,J=6.3, 12.6Hz,1H); 6.8-6.95(m,2H); 7-7.1(m,3H); 7.7(d,J=8.7Hz,2H) |
| 81 | 113-5 | 1508, 1315, 1155, 1133, 1067, 831 | 2.9(dd,J=8.4, 17.4Hz,1H); 3(s,3H); 3.7(dd,J= 12.6, 17.4Hz,1H); 5.4(dd,J=8.4, 12.6Hz,1H); 6.9(m,4H); 7.45(d,J=8.4Hz,2H); 7.95(d,J=8.4 Hz,2H) |
| 82 | 113-4 | 1508, 1315, 1155, 1133, 1067, 827 | 2.9(dd,J=8.4, 17.4Hz,1H); 3(s,3H); 3.7(dd,J= 12.6, 17.4Hz,1H); 5.4(dd,J=8.4, 12.6Hz,1H); 6.9(m,4H); 7.45(d,J=8.4Hz,2H); 7.95(d,J=8.4 Hz,2H) |
| 83 | 86-9 | 1596, 1510, 1314, 1264, 1150, 845 | 3(s+dd, 4H); 3.6 (dd, J=12.2, 17,6Hz, 1H) ; 5.6 (dd, J=6.2, 12.2Hz, 1H); 6.65 (t, J=9Hz, 1H) ; 6.75 (t, J=8Hz, 1H) ; 7,35 (m, 3H) ; 7.8 (d, J=8.3Hz, 2H) |
| 84 | 84-6 | 1596, 1510, 1314, 1264, 1150, 845 | 3(s+dd, 4H); 3.6 (dd, J=12.2, 17.6Hz, 1H); 5.6 (dd, J=6.2, 12,2Hz, 1H); 6.65 (t, J=9Hz, 1H); 6.75 (t, J=8Hz, 1H); 7.35 (m, 3H); 7.8 (d, J=8.3Hz, 2H) |

In human therapy, the administration dose of the compounds of the present invention depends on the seriousness of the condition to be treated. It will normally lie between 10 and 500 mg/day. The compounds of the invention can be administered as a single active principle or together with another product, in order to obtain a synergy. The compounds of the invention, with a suitable pharmacological formulation, may be given orally, transdermically, parenterally, subcutaneously, intranasally, intramuscularly or intravenously. Pharmaceutical compositions containing the compounds of general formula (I) are described in our patent application WO 99/62884.

### Biological evaluation

The compounds with the general formula (I) are useful for treatment of preneoplasic or neoplasic processes, angiogenesis, cachexia and processes related to tumour necrosis factor (TNF) and, in general, processes that can benefit from inhibition of the expression of the gene in charge of the synthesis of cyclooxygenase-2 (COX-2). In order to demonstrate these activities below are provided, by way of example, several pharmacological tests.

### Inhibition of the induction of COX-2

In order to determine the inhibition capability of the transcriptional induction of cyclooxygenase-2 a cell system was used of stable transfectant JURKAT cells with the promoter of the gene COX-2 associated to the gene for luciferase. Three independent clones were used (C3, C7 and F9) which differ in their basal luciferase activity ranging from 4500 to 180.000 RLUs/10⁵ cells, which are increased as a response to stimulant agents such as protoinflammatory type activators of the cell signalling paths as forbol esters (TPA) and ionofors (ionomycine).

For the test the cells were pre-treated for 1 hour with the compounds to test and then stimulated with TPA + ionomycine for 6 hours. Cell extracts were then obtained in which the luciferase activity was examined and the protein concentration determined. Inhibition of the luciferase activity gives an indication of the inhibition of the induction of COX-2.

As an example of the activity of the compounds with general formula (I), we shown below the results obtained with the compound of Example 10, which show a clear inhibition of the induction of COX-2.

| Example 10 | Inhibition of the activity of luciferase (%) | | |
|---|---|---|---|
| (Concentration µg/ml) | | | |
| | Clone C7 | Clone C9 | Clone F9 |
| 0.5 | 20% | 40% | 32% |
| 5 | 55% | 75% | 65% |
| 50 | 85% | 85% | 95% |
| IC-50 (µg/ml) | 5.8 | 0.9 | 1.8 |

### Anti-tumoral activity in the human colon carcinoma

The anti-tumoral activity of the compounds with general formula (I) was studied, determining their effect on cell lines of human colon carcinoma (TD 20 and NC 59). The two cell lines have the wild protein K-Ras. TD20 contains a mutation of the p53 suppressor gene, while NC59 has the p53 wild protein.

Both cell lines were cultured in a DMEM medium (Life Technologies) supplemented with 10% foetal bovine serum (Life Technologies) at 37°C and 5% CO₂.

Cytotoxicity tests were carried out using the XTT kit (Boehringer-Manheim) which measures the cell capability to metabolise a tetrazoyl salt to formazan.

| Concentration | (µM) | % Viability of colorectal cancer cells ± SD | |
|---|---|---|---|
| | | NC59 | TD 20 |
| Example 3 | 1 | 99.64 ± 0.51 | 98.58 ± 2.83 |
| | 20 | 76.52 ± 8.70 | 84.96 ± 3.05 |
| | 30 | 56.50 ± 0.25 | 66.34 ± 5.84 |
| | 40 | 16.74 ±10.53 | 27.38 ± 4.12 |
| | 60 | 1.16 ± 0.83 | 1.79 ± 2.09 |
| | 100 | 0 ± 0 | 0.69 ± 0.01 |
| | | IC-50 = 29.87 | IC-50 = 33.87 |
| Example 10 | 1 | 92.09 ± 9.35 | 95.03 ± 8.87 |
| | 20 | 76.14 ± 7.17 | 90.24 ± 5.97 |
| | 40 | 15.77 ± 9.37 | 43.13 ±13.77 |
| | 60 | 2.59 ± 1.54 | 5.42 ± 4.11 |
| | 80 | 1.66 ± 1.57 | 3.22 ± 3.46 |
| | | IC-50 = 27.18 µM | IC-50 = 37.92 µM |

As an example of the activity of the compounds with general formula (I), the compounds of examples 3 and 10 showed cytotoxic activity on both human colon carcinoma cell lines. Their IC50 on cell line NC59 was 30µM and 27µM and on line TD20 it was 34 µM and 38 µM, respectively.

In order to obtain a deeper knowledge of the action mechanism of these compounds the capability to induce apoptosis in the tumour cell line TD20 of the compound of example 3 was studied (dose 100 µM). In these conditions 20% apoptotic cells were found after 24h and 80% after 48h, while in the cells treated with a carrier the apoptotic cells did not exceed 1%. Cytotoxicity levels, measured by XTT, were similar to apoptosis levels, suggesting that the cytotoxicity activity of example 3 is due to their ability to induce apoptosis.

The signal transduction paths associated to the apoptosis process were then studied. After exposing NC59 cells to a 100 µM concentration of the compound of example 3, the expression levels were studied for p53, FAK and β-actine and the activation level of MAP kinases, JNK and PKB/Akt, after 5 and 20 hours of exposure. The results demonstrated the activation of signal transduction paths other than p53, JNK or p38, which are the paths activated by conventional genotoxic drugs (e.g. 5-FU).

In summary, it can be said that the compounds of examples 3 and 10 are effective anti-tumoral agents for human colon carcinoma, which suggests the possibility of using the compounds with general formula (I) not only as chemopreventives but in established colon cancer. In addition, their anti-tumoral effect is mediated by apoptosis and the activation of the transduction paths is effected by signals other than p53, JNK or p38, which suggests that these compounds may constitute an addition to current chemotherapy protocols as they have a different cytotoxic action mechanism.

### Anti-tumoral activity in mammary carcinoma

The anti-tumoral activity of the compounds with general formula (I) was studied, determining their effect on a mammary carcinoma cell line(MDAMB 453). The cell line was cultured in a DMEM medium (Life Technologies) supplemented with 10% foetal bovine serum (Life Technologies) at 37°C and 5% CO₂.

Cytotoxicity tests were carried out using the XTT kit (Boehringer-Manheim) which measures the cell capability to metabolise a tetrazoyl salt to formazan.

| Concentration | (µM) | % Viability of mammary cancer cells ± SD |
|---|---|---|
| | | MDAMB 453 |
| Example 3 | 1 | 94.76 ± 8.71 |
| | 20 | 31.52 ± 9.12 |
| | 40 | 12.77 ± 4.80 |
| | 60 | 5.97 ± 5.06 |
| | 80 | 2.84 ± 1.71 |
| | | IC-50 = 12.87 µM |
| Example 10 | 1 | 97.53 ± 3.25 |
| | 20 | 42.75 ± 9.31 |
| | 40 | 33.30 ± 7.34 |
| | 60 | 8.25 ± 6.82 |
| | 80 | 5.8± 4.76 |
| | | IC-50 = 17.88 µM |

As an example of the activity of the compounds with general formula (I), the compounds of Examples 3 and 10 showed cytotoxic activity on the mammary carcinoma cell line, with an IC50 of 3µM (Example 3) and 18 µM (Example 10).

In summary, it can be said that the compounds of examples 3 and 10 are effective anti-tumoral agents for mammary carcinoma, which suggests the possibility of using the compounds with general formula (I) not only as chemopreventives but in established mammary carcinoma.

### Antiangiogenic activity

This activity has been studied determining the inhibition of the induction of the expression of VEGF. The increased expression of vascular-endothelial growth factor (VEGF) has been related to tumour progression and angiogenesis. VEGF is a proangiogenic factor, promoting mitogenesis, migration and an increased vascular permeability of in vitro endothelial cells. It has been shown recently that COX-2 can regulate the angiogenesis process induced by colon cancer cells, increasing the expression of proangiogenic factors by said cells. Inhibition of COX-2 may block this process, inhibiting the expression of some of these factors, such as VEGF.

In turn, VEGF also induces the expression of Tissue Factor (TF) in monocyte membranes, epithelial cells and endothelium. Although the main function of TF is to initiate the coagulation cascade, it can transduce intracellular signals participating in metastasis and angiogenesis associated to tumours. TF facilitates in vivo growth of the tumour, favouring angiogenesis. It has been demonstrated that tumour cells transfected with TF produce greater vascularization.

In order to study the antiangiogenic activity of the products with general formula (I), the expression of the VEGF promoter was studied in a basal and stimulated situation using the Caco-2 human colon carcinoma cell line, temporally transfected with a vector containing the promoter f the human VEGF gene. Once the test conditions were established the capability of the product studied to inhibit the expression of the VEGF and TF genes was studied by measuring the luciferase activity of their promoters.

In the test 1.25 x 10⁵ Caco-2 cells were used temporally transfected with t corresponding DNA in 500 µl of DEMEM-10% FCS medium in 24 dimple plates. The cells were pre-treated for 1 hour with the compounds to be tested, and later stimulated with TPA for 16 hours. Afterwards cell extracts were obtained and their luciferase activity examined, determining the protein concentration.

By way of example of the activity of the compounds with general formula (I), below are provided the results obtained for the compounds f examples 3 and 10, which show a clear antiangiogenic effect as both VEGF and TF are inhibited.

| Compound | Inhibition of the induction of | |
|---|---|---|
| | VEGF | TF |
| | IC-50 (µg/ml) | IC-50 (µg/ml) |
| Example 3 | 20 | 25 |
| Example 10 | 25 | 22 |

### Inhibition of tumour necrosis factor -α (TNF-α)

Tumour necrosis factor -α (TNF-α) is a cytokine, a powerful proinflammatory and immunomodulator involved in various inflammatory conditions such as rheumatoid arthritis, CROHN's disease, multiple sclerosis and cachexia associated with cancer, as well as in human immunodeficiency associated to viral infections. TNF-α was originally described due to its capacity to induce haemorrhagic necrosis of certain tumours in animals treated with lipopolysaccharide (LPS). It was also called cachectin, as it is a circulating mediator of the wear syndrome related to certain parasitary diseases. Cachexia, or the wear resulting from TNF, is related to its property of increasing the lipase of lipoprotein and for this reason exhausts adipose cells. TNF-α is produced predominantly by macrophages when activated by a great variety of stimuli, such as the presence of bacterial or mycobacterial proteins, fungal antigens, virus, C5a complement or gamma interferon.

TNF-α is one of the mediators of Gram- bacterial endotoxic shock, and seems to be responsible for fever, metabolic acidosis, diarrhoea, hypertension, disseminated vascular coagulation and in certain cases even death. In addition, TNF-α can lead to the activation of neutrophiles, inducing the genic expression of IL-1, increase the expression of MHC antigens (with Class I major histocompatibility) in endothelial cells and is involved in reabsorbtion of the bone marrow and in production of PGE₂ and collagenase of synovial cells and human fibroblasts. Thus, products capable of antagonising the activity of this mediator can have clinical value to combat their lethal effects *(C.A. Mc Intyre et al: Drugs News and Perspectives 1992 5 (4): 207-213; A.J.H. Gearing et al: Nature 1994 370: 555-557; M.A. Pahlavani: Drugs of Today 1993 29 (8):* 525-533).

The study of the inhibitory activity of TNF-α has been performed according to the method described by P. Klemen et al. *(Europ. J. Pharmacol. 1995 281: 69-74)*, consisting of determining the production of TNF-α in the localised area where the inflammation is acute, specifically using the zymosan inflated air pouch model in mice. The TNF-α present in the inflammatory exudates produced in said pouch as a result of the stimulation with zymosan was determined. The analytical determination of TNF-α was performed by ELISA.
by way of example of the activity of the compounds with general formula (I), below are shown the results obtained with the compound of Example 10, which shows a considerable TNF-α inhibitory activity, very well correlated with the dose.

| Example 10 Doses (mg/kg, i.p.) | Air pouch model in mice % Inhibition of TNF-α in inflammatory exudate stimulated by zymosan |
|---|---|
| 0.039 | 12.6 ± 2.6 |
| 0.156 | 15.7 ± 4.9 |
| 0.625 | 46.8 ± 12.5 |
| 2.5 | 58.8 ± 13.6 |
| DE-50 = 1.20 mg/kg, i.p. | |
| (r = 0.957) (*) | |

i.p.: intraperitoneal

## Claims

1. Use of a derivative of pyrazoline with the general formula (I) 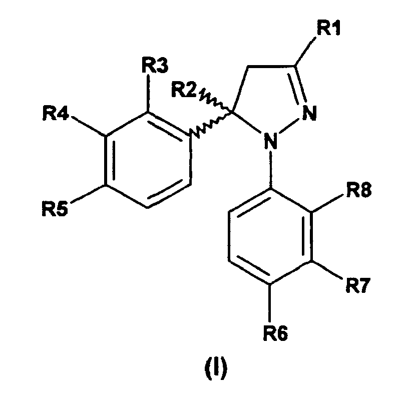 wherein
R₁ represents an atom of hydrogen, a methyl, fluoromethyl, difluoromethyl, trifluoromethyl group, carboxylic acid, lower alkyl carboxylate with 1 to 4 carbon atoms, carboxamide or cyano group,
R₂ represents an atom of hydrogen or a methyl group,
R₃, R₄, R₇ and R₈, like or different, represent an atom of hydrogen, chlorine, fluorine, a methyl, trifluoromethyl or methoxy group,
R₅ represents an atom of hydrogen, chlorine, fluorine, a methyl, trifluoromethyl, methoxy, trifluoromethoxy, methylsulphonyl, aminosulphonyl or acetylaminosulphonyl group,
R₆ represents an atom of hydrogen, chlorine, fluorine, a methyl, trifluoromethyl, methoxy, trifluoromethoxy, methylsulphonyl, group,
with the condition that one of the substituents R₅ or R₆ is a methylsulphonyl, aminosulphonyl or acetylaminosulphonyl group, and
with the condition that when R₁ represents a methyl group
R₂ represents an atom of hydrogen or a methyl group,
R₃ and R₈, like or different, represent an atom of hydrogen, chlorine, fluorine, a methyl or trifluoromethyl group,
R₄ represents an atom of hydrogen, fluorine, a methyl, trifluoro-methyl or methoxy group,
R₅ represents a fluorine atom, a trifluoromethyl, trifluoromethoxy, methylsulphonyl, aminosulphonyl or acetylaminosulphonyl group,
R₆ represents an atom of hydrogen, chlorine, fluorine, a methyl, trifluoromethyl, methoxy, trifluoromethoxy, methylsulphonyl, group,
with the condition that one of the substituents R₅ or R₆ is a methylsulphonyl, aminosulphonyl or acetylaminosulphonyl group, and
R₇ represents an atom of hydrogen, chlorine, fluorine, a methyl, trifluoromethyl or methoxy group; Or one of their physiologically acceptable salts, in the preparation of a medicament for preventing or treating cell proliferation diseases, particularly for preventing or treating preneoplasic or neoplasic processes, tumoral angiogenensis, cachexia and processes related to tumour necrosis factor (TNF) and, in general, processes that can benefit from inhibition of the expression of the gene responsible for synthesising cyclooxygenase-2 (COX-2) in mammals, including man.

2. Use according to claim 1 of a compound with the general formula (I) selected from among the group comprised of:
[4] 4,5-Dihydro-1-(4-methylphenyl)-5-(4-methylsulphonylphenyl) -3-trifluoromethyl-1*H*- pyrazol
[6] 4,5-Dihydro-5-phenyl-1-(4-methylsulphonylphenyl)-3-trifluoromethyl-1*H*-pyrazol
[7] 4,5-Dihydro-5-(4-methylphenyl)-1-(4-methylsulphonylphenyl) -3-trifluoromethyl-1*H*- pyrazol
[9] 4,5-Dihydro-5-(4-fluorophenyl)-1-(4-methylsulphonylphenyl)-3-trifluoromethyl-1*H*-pyrazol
[10] 4,5-Dihydro-1-(4-fluorophenyl)-5-(4-methylsulphonylphenyl)-3-trifluoromethyl-1*H*-pyrazol
[12] 5-(2,4-Dichlorophenyl)-4,5-dihydro-1-(4-methylsulphonylphenyl)-3-trifluoromethyl-1*H*-pyrazol
[17] 4,5-Dihydro-5-(2-fluorophenyl)-1-(4-methylsulphonylphenyl)-3-trifluoromethyl-1*H*- pyrazol
[19] 4,5-Dihydro-5-(3-fluorophenyl)-1-(4-methylsulphonylphenyl)-3-trifluoromethyl-1H- pyrazol
[25] 5-(3,4-Difluorophenyl)-4,5-dihydro-1-(4-methylsulphonylphenyl)-3-trifluoromethyl-1*H*-pyrazol
[27] 4,5-Dihydro-5-(4-fluorophenyl)-3-methyl-1-(4-methylsulphonylphenyl)-1*H*-pyrazol
[29] 4,5-Dihydro-3-methyl-5-(4-methylphenyl)-1-(4-methylsulphonylphenyl) -1*H*-pyrazol
[32] 4,5-Dihydro-5-phenyl-1-(4-methylsulphonylphenyl) -1*H*-pyrazol
[33] 4,5-Dihydro-3-methyl-1-(4-methylsulphonylphenyl)-5-(4-trifluoromethylphenyl)-1*H*- pyrazol
[36] 4,5-dihydro-5-(4-methylphenyl)-1-(4-methylsulphonylphenyl)-1*H*-pyrazol-3-carboxylic acid
[39] 4,5-Dihydro-5-(4-methylphenyl)-1-(4-methylsulphonylphenyl)-1*H*-pyrazol-3-methyl carboxylate
[42] 4,5-Dihydro-5-(4-methylphenyl)-1-(4-methylsulphonylphenyl)-1*H*-pyrazol-3- carboxamide
[43] 3-Cyano-4,5-dihydro-5-(4-methylphenyl)-1-(4-methylsulphonylphenyl)-1*H*-pyrazol
[59] 1-(2,4-Difluorophenyl)-4,5-dihydro-5-(4-methylsulphonylphenyl)-3-trifluoromethyl-1*H*- pyrazol
[65] 1-(4-Chlorophenyl)-4,5-dihydro-5-(4-methylsulphonylphenyl)-3-trifluoromethyl-1H- pyrazol
[66] 4,5-Dihydro-1-phenyl-5-(4-methylsulphonylphenyl)-3-trifluoromethyl-1*H*-pyrazol
[67] 4,5-Dihydro-1-(2-fluorophenyl)-5-(4-methylsulphonylphenyl)-3-trifluoromethyl-1H- pyrazol
[68] 1-(4-Chloro-2-methylphenyl)-4,5-dihydro-5-(4-methylsulphonylphenyl)-3-trifluoromethyl-1*H*-pyrazol
[69] 4,5-Dihydro-1-(3-fluorophenyl)-5-(4-methylsulphonylphenyl)-3-trifluoromethyl-1*H*- pyrazol
[70] 4,5-Dihydro-1-(3-methylphenyl)-5-(4-methylsulphonylphenyl)-3-trifluoromethyl-1*H*- pyrazol
[71] 4,5-Dihydro-1-(2,4-dimethylphenyl)-5-(4-methylsulphonylphenyl)-3-trifluoromethyl-1*H*- pyrazol
[72] 1-(2-Chlorophenyl)-4,5-dihydro-5-(4-methylsulphonylphenyl)-3-trifluoromethyl-1*H*- pyrazol
[73] 4,5-Dihydro-1-(2-methylphenyl)-5-(4-methylsulphonylphenyl)-3-trifluoromethyl-1*H*- pyrazol
[74] 1-(2,4-Dichlorophenyl)-4,5-dihydro-5-(4-methylsulphonylphenyl)-3-trifluoromethyl-1*H*- pyrazol
[75] 4,5-Dihydro-5-(4-methylsulphonylphenyl)-3-trifluoromethyl-1-(2-trifluoromethyl phenyl)-1*H*-pyrazol
[76] 5-(4-Aminosulphonylphenyl)-4,5-Dihydro-1-(4-fluorophenyl)-3-trifluoromethyl-1*H*-pyrazol
[77] 4,5-Dihydro-1-phenyl-3-methyl-5-(4-methylsulphonylphenyl)-1*H*-pyrazol
[78] 4,5-Dihydro-1-(4-fluorophenyl)-5-(4-methylsulphonylphenyl)-1*H*-pyrazol
[81](+)-4,5-Dihydro-1-(4-fluorophenyl)-5-(4-methylsulphonylphenyl)-3-trifluoromethyl-1*H*- pyrazol
[82](-)-4,5-Dihydro-1-(4-fluorophenyl)-5-(4-methylsulphonylphenyl)-3-trifluoromethyl-1*H*- pyrazol
[83](+)-1-(2,4-Difluorophenyl)-4,5-dihydro-5-(4-methylsulphonylphenyl)-3-trifluoromethyl-1*H*-pyrazol
[84](-)-1-(2,4-Difluorophenyl)-4,5-dihydro-5-(4-methylsulphonylphenyl)-3-trifluoromethyl-1*H*-pyrazol;
or one of their physiologically acceptable salts, in the preparation of a medicament for preventing or treating cell proliferation diseases, particularly for preventing or treating preneoplasic or neoplasic processes, tumoral angiogenensis, cachexia and processes related to tumour necrosis factor (TNF) and, in general, those processes that can benefit from inhibition of the expression of the gene responsible for synthesising cyclooxygenase-2 (COX-2) in mammals, including man.

3. Use according to claim 1 of a compound with the general formula (I), or one of its physiologically acceptable salts, together with another product commonly used in the treatment of neoplasias, producing in this case a synergy, in the preparation of a medicament for preventing or treating cell proliferation diseases, particularly for preventing or treating preneoplasic or neoplasic processes, tumoral angiogenensis, cachexia and processes related to tumour necrosis factor (TNF) and, in general, processes that can benefit from inhibition of the expression of the gene responsible for synthesising cyclooxygenase-2 (COX-2) in mammals, including man.

## Patentansprüche

1. Verwendung eines Pyrazolinderivats mit der allgemeinen Formel (I) 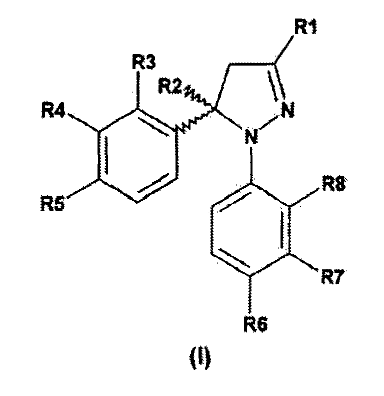 worin
R₁ ein Wasserstoffatom, eine Methyl-, Fluormethyl-, Difluormethyl-, Trifluormethylgruppe, Carbonsäure, niederes Alkylcarboxylat mit 1 bis 4 Kohlenstoffatomen, Carboxamid- oder Cyanogruppe bedeutet,
R₂ ein Wasserstoffatom oder eine Methylgruppe bedeutet,
R₃, R₄, R₇ und R₈, die gleich oder voneinander verschieden sind, ein Wasserstoffatom, Chloratom, Fluoratom, eine Methyl-, Trifluormethyl- oder Methoxygruppe bedeuten,
R₅ ein Wasserstoffatom, Chloratom, Fluoratom, eine Methyl-, Trifluormethyl-, Methoxy-, Trifluormethoxy-, Methylsulfonyl-, Aminosulfonyl- oder Acetylaminosulfonylgruppe bedeutet,
R₆ ein Wasserstoffatom, Chloratom, Fluoratom, eine Methyl-, Trifluormethyl-, Methoxy-, Trifluormethoxy-, Methylsulfonylgruppe bedeutet,
mit der Maßgabe, dass einer der Substituenten R₅ oder R₆ eine Methylsulfonyl-, Aminosulfonyl- oder Acetylaminosulfonylgruppe ist, und
mit der Maßgabe, dass, wenn R₁ eine Methylgruppe bedeutet
R₂ ein Wasserstoffatom oder eine Methylgruppe bedeutet,
R₃ und R₈, die gleich oder voneinander verschieden sind, ein Wasserstoffatom, Chloratom, Fluoratom, eine Methyl- oder Trifluormethylgruppe bedeuten,
R₄ ein Wasserstoffatom, Fluoratom, eine Methyl-, Trifluormethyl- oder Methoxygruppe bedeutet,
R₅ ein Fluoratom, eine Trifluormethyl-, Trifluormethoxy-, Methylsulfonyl-, Aminosulfonyl- oder Acetylaminosulfonylgruppe bedeutet,
R₆ ein Wasserstoffatom, Chloratom, Fluoratom, eine Methyl-, Trifluormethyl-, Methoxy-, Trifluormethoxy-, Methylsulfonylgruppe bedeutet,
mit der Maßgabe, dass einer der Substituenten R₅ oder R₆ eine Methylsulfonyl-, Aminosulfonyl- oder Acetylaminosulfonylgruppe ist, und
R₇ ein Wasserstoffatom, Chloratom, Fluoratom, eine Methyl-, Trifluormethyl- oder Methoxygruppe bedeutet;
oder eines ihrer physiologisch annehmbaren Salze,
bei der Herstellung eines Medikaments zum Verhüten oder Behandeln von Zellproliferationskrankheiten, insbesondere zum Verhüten oder Behandeln von präneoplastischen oder neoplastischen Prozessen, Tumorangiogenese, Kachexie und Prozessen, die mit dem Tumornekrosefaktor (TNF) zusammenhängen, und allgemein Prozessen, welche aus der Hemmung der Expression des für die Synthese von Cyclooxygenase-2 (COX-2) verantwortlichen Gens Nutzen ziehen können, bei Säugem, einschließlich des Menschen.

2. Verwendung nach Anspruch 1 einer Verbindung mit der allgemeinen Formel (I), ausgewählt aus der Gruppe umfassend:
[4] 4,5-Dihydro-1-(4-methylphenyl)-5-(4-methylsulfonylphenyl)-3-trifluormethyl-1H-pyrazol
[6] 4,5-Dihydro-5-phenyl-1-(4-methylsulfonylphenyl)-3-trifluormethyl-1H-pyrazol
[7] 4,5-Dihydro-5-(4-methylphenyl)-1-(4-methylsulfonylphenyl)-3-trifluormethyl-1H-pyrazol
[9] 4,5-Dihydro-5-(4-fluorphenyl)-1-(4-methylsulfonylphenyl)-3-trifluormethyl-1H-pyrazol
[10] 4,5-Dihydro-1-(4-fluorphenyl)-5-(4-methylsulfonylphenyl)-3-trifluormethyl-1H-pyrazol
[12] 5-(2,4-Dichlorphenyl)-4,5-dihydro-1-(4-methylsulfonylphenyl)-3-trifluormethyl-1 H-pyrazol
[17] 4,5-Dihydro-5-(2-fluorphenyl)-1-(4-methylsulfonylphenyl)-3-trifluormethyl-1H-pyrazol
[19] 4,5-Dihydro-5-(3-fluorphenyl)-1-(4-methylsulfonylphenyl)-3-trifluormethyl-1H-pyrazol
[25] 5-(3,4-Difluorphenyl)-4,5-dihydro-1-(4-methylsulfonylphenyl)-3-trifluormethyl-1 H-pyrazol
[27] 4,5-Dihydro-5-(4-fluorphenyl)-3-methyl-1-(4-methylsulfonylphenyl)-1H-pyrazol
[29] 4,5-Dihydro-3-methyl-5-(4-methylphenyl)-1-(4-methylsulfonylphenyl)-1H-pyrazol
[32] 4,5-Dihydro-5-phenyl-1-(4-methylsulfonylphenyl)-1H-pyrazol
[33] 4,5-Dihydro-3-methyl-1-(4-methylsulfonylphenyl)-5-(4-trifluormethylphenyl)-1 H-pyrazol
[36] 4,5-Dihydro-5-(4-methylphenyl)-1-(4-methylsulfonylphenyl)-1H-pyrazol-3-carbonsäure
[39] 4,5-Dihydro-5-(4-methylphenyl)-1-(4-methylsulfonylphenyl)-1H-pyrazol-3-methylcarboxylat
[42] 4,5-Dihydro-5-(4-methylphenyl)-1-(4-methylsulfonylphenyl)-1H-pyrazol-3-carboxamid
[43] 3-Cyano-4,5-dihydro-5-(4-methylphenyl)-1-(4-methylsulfonylphenyl)-1H-pyrazol
[59] 1-(2,4-Difluorphenyl)-4,5-dihydro-5-(4-methylsulfonylphenyl)-3-trifluormethyl-1H-pyrazol
[65] 1-(4-Chlorphenyl)-4,5-dihydro-5-(4-methylsulfonylphenyl)-3-trifluormethyl-1H-pyrazol
[66] 4,5-Dihydro-1-phenyl-5-(4-methylsulfonylphenyl)-3-trifluormethyl-1H-pyrazol
[67] 4,5-Dihydro-1-(2-fluorphenyl)-5-(4-methylsulfonylphenyl)-3-trifluormethyl-1H-pyrazol
[68] 1-(4-Chlor-2-methylphenyl)-4,5-dihydro-5-(4-methylsulfonylphenyl)-3-trifluormethyl-1H-pyrazol
[69] 4,5-Dihydro-1-(3-fluorphenyl)-5-(4-methylsulfonylphenyl)-3-trifluormethyl-1H-pyrazol
[70] 4,5-Dihydro-1-(3-methylphenyl)-5-(4-methylsulfonylphenyl)-3-trifluormethyl-1 H-pyrazol
[71] 4,5-Dihydro-1-(2,4-dimethylphenyl)-5-(4-methylsulfonylphenyl)-3-trifluormethyl-1H-pyrazol
[72] 1-(2-Chlorphenyl)-4,5-dihydro-5-(4-methylsulfonylphenyl)-3-tritluormethyl-1H-pyrazol
[73] 4,5-Dihydro-1-(2-methylphenyl)-5-(4-methylsulfonylphenyl)-3-trifluormethyl-1 H-pyrazol
[74] 1-(2,4-Dichlorphenyl)-4,5-dihydro-5-(4-methylsulfonylphenyl)-3-trifluormethyl-1 H-pyrazol
[75] 4,5-Dihydro-5-(4-methylsulfonylphenyl)-3-trifluormethyl-1-(2-trifluormethylphenyl)-1H-pyrazol
[76] 5-(4-Aminosulfonylphenyl)-4,5-dihydro-1-(4-fluorphenyl)-3-trifluormethyl-1H-pyrazol
[77] 4,5-Dihydro-1-phenyl-3-methyl-5-(4-methylsulfonylphenyl)-1H-pyrazol
[78] 4,5-Dihydro-1-(4-fluorphenyl)-5-(4-methylsulfonylphenyl)-1H-pyrazol
[81] (+)-4,5-Dihydro-1-(4-fluorphenyl)-5-(4-methylsulfonylphenyl)-3-trifluormethyl-1 H-pyrazol
[82] (-)-4,5-Dihydro-1-(4-fluorphenyl)-5-(4-methylsulfonylphenyl)-3-trifluormethyl-1 H-pyrazol
[83] (+)-1-(2,4-Difluorphenyl)-4,5-dihydro-5-(4-methylsulfonylphenyl)-3-trifluormethyl-1 H-pyrazol
[84] (-)-1-(2,4-Difluorphenyl)-4,5-dihydro-5-(4-methylsulfonylphenyl)-3-trifluormethyl-1 H-pyrazol;
oder eines ihrer physiologisch annehmbaren Salze,
bei der Herstellung eines Medikaments zum Verhüten oder Behandeln von Zellproliferationskrankheiten, insbesondere zum Verhüten oder Behandeln von präneoplastischen oder neoplastischen Prozessen, Tumorangiogenese, Kachexie und Prozessen, die mit dem Tumornekrosefaktor (TNF) zusammenhängen, und allgemein solchen Prozessen, welche aus der Hemmung der Expression des für die Synthese von Cyclooxygenase-2 (COX-2) verantwortlichen Gens Nutzen ziehen können, bei Säugern, einschließlich des Menschen.

3. Verwendung nach Anspruch 1 einer Verbindung mit der allgemeinen Formel (I) oder eines ihrer physiologisch annehmbaren Salze, zusammen mit einem anderen Produkt, das üblicherweise bei der Behandlung von Neoplasien verwendet wird, wobei in diesem Fall eine Synergie auftritt, bei der Herstellung eines Medikaments zum Verhüten oder Behandeln von Zellproliferationskrankheiten, insbesondere zum Verhüten oder Behandeln von präneoplastischen oder neoplastischen Prozessen, Tumorangiogenese, Kachexie und Prozessen, die mit dem Tumornekrosefaktor (TNF) zusammenhängen, und allgemein Prozessen, welche aus der Hemmung der Expression des für die Synthese von Cyclooxygenase-2 (COX-2) verantwortlichen Gens Nutzen ziehen können, bei Säugern, einschließlich des Menschen.

## Revendications

1. Utilisation d'un dérivé de pyrazoline de formule générale (I) : 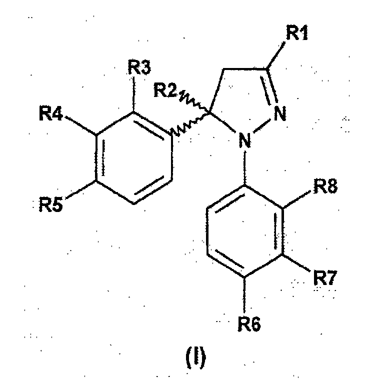 dans laquelle
R₁ représente un atome d'hydrogène, un groupe méthyle, fluorométhyle, difluorométhyle, trifluorométhyle, un acide carboxylique, un carboxylate d'alkyle inférieur ayant de 1 à 4 atomes de carbone, un groupe carboxamide ou cyano,
R₂ représente un atome d'hydrogène ou un groupe méthyle,
R₃, R₄, R₇ et R₈, identiques ou différents, représentent un atome d'hydrogène, de chlore, de fluor, un groupe méthyle, trifluorométhyle ou méthoxy,
R₅ représente un atome d'hydrogène, de chlore, de fluor, un groupe méthyle, trifluorométhyle, méthoxy, trifluorométhoxy, méthylsulfonyle, aminosulfonyle ou acétylaminosulfonyle,
R₆ représente un atome d'hydrogène, de chlore, de fluor, un groupe méthyle, trifluorométhyle, méthoxy, trifluorométhoxy, méthylsulfonyle,
avec comme condition que l'un des substituants R₅ et R₆ est un groupe méthylsulfonyle, aminosulfonyle ou acétylaminosulfonyle, et
avec comme condition que, lorsque R₁ représente un groupe méthyle,
R₂ représente un atome d'hydrogène ou un groupe méthyle,
R₃ et R₈, identiques ou différents, représentent un atome d'hydrogène, de chlore, de fluor, un groupe méthyle ou trifluorométhyle,
R₄ représente un atome d'hydrogène, de fluor, un groupe méthyle, trifluorométhyle ou méthoxy,
R₅ représente un atome de fluor, un groupe trifluorométhyle, trifluorométhoxy, méthylsulfonyle, aminosulfonyle ou acétylaminosulfonyle,
R₆ représente un atome d'hydrogène, de chlore, de fluor, un groupe méthyle, trifluorométhyle, méthoxy, trifluorométhoxy, méthylsulfonyle,
avec comme condition que l'un des substituants R₅ ou R₆ est un groupe méthylsulfonyle, aminosulfonyle ou acétylaminosulfonyle, et
R₇ représente un atome d'hydrogène, de chlore, de fluor, un groupe méthyle, trifluorométhyle ou méthoxy ;
ou de l'un de ses sels physiologiquement acceptables,
dans la préparation d'un médicament pour prévenir ou traiter des maladies de prolifération cellulaire, en particulier pour prévenir ou traiter des processus pré-néoplasiques ou néoplasiques, une angiogenèse tumorale, une cachexie et des processus liés au facteur nécrosant les tumeurs (TNF) et, en général, des processus qui peuvent tirer avantage d'une inhibition de l'expression du gène responsable de la synthèse de la cyclooxygénase-2 (COX-2) chez les mammifères, y compris l'homme.

2. Utilisation selon la revendication 1 d'un composé de formule générale (I) choisi dans le groupe constitué par les suivants :
[4] 4,5-dihydro-1-(4-méthylphényl)-5-(4-méthylsulfonylphényl)-3-trifluorométhyl-1H-pyrazole
[6] 4,5-dihydro-5-phényl-1-(4-méthylsulfonylphényl)-3-trifluorométhyl-1H-pyrazole
[7] 4,5-dihydro-5-(4-méthylphényl)-1-(4-méthylsulfonylphényl)-3-trifluorométhyl-1H-pyrazole
[9] 4,5-dihydro-5-(4-fluorophényl)-1-(4-méthylsulfonylphényl)-3-trifluorométhyl-1H-pyrazole
[10] 4,5-dihydro-1-(4-fluorophényl)-5-(4-méthylsulfonylphényl)-3-trifluorométhyl-1H-pyrazole
[12] 5-(2,4-dichlorophényl)-4,5-dihydro-1-(4-méthylsulfonylphényl)-3-trifluorométhyl-1H-pyrazole
[17] 4,5-dihydro-5-(2-fluorophényl)-1-(4-méthylsulfonylphényl)-3-trifluorométhyl-1H-pyrazole
[19] 4,5-dihydro-5-(3-fluorophényl)-1-(4-méthylsulfonylphényl)-3-trifluorométhyl-1H-pyrazole
[25] 5-(3,4-difluorophényl)-4,5-dihydro-1-(4-méthylsulfonylphényl)-3-trifluorométhyl-1H-pyrazole
[27] 4,5-dihydro-5-(4-fluorophényl)-3-méthyl-1-(4-méthylsulfonylphényl)-1H-pyrazole
[29] 4,5-dihydro-3-méthyl-5-(4-méthylphényl)-1-(4-méthylsulfonylphényl)-1H-pyrazole
[32] 4,5-dihydro-5-phényl-1-(4-méthylsulfonylphényl) -1H-pyrazole
[33] 4,5-dihydro-3-méthyl-1-(4-méthylsulfonylphényl)-5-(4-trifluorométhylphényl)-1H-pyrazole
[36] acide 4,5-dihydro-5-(4-méthylphényl)-1-(4-méthylsulfonylphényl)-1H-pyrazol-3-carboxylique
[39] carboxylate de 4,5-dihydro-5-(4-méthylphényl)-1-(4-méthylsulfonylphényl)-1H-pyrazol-3-méthyle carboxylate
[42] 4,5-dihydro-5-(4-méthylphényl)-1-(4-méthylsulfonylphényl)-1H-pyrazol-3-carboxamide
[43] 3-cyano-4,5-dihydro-5-(4-méthylphényl)-1-(4-méthylsulfonylphényl)-1H-pyrazole
[59] 1-(2,4-difluorophényl)-4,5-dihydro-5-(4-méthylsulfonylphényl)-3-trifluorométhyl-1H-pyrazole
[65] 1-(4-chlorophényl)-4,5-dihydro-5-(4-méthylsulfonylphényl)-3-trifluorométhyl-1H-pyrazole
[66] 4,5-dihydro-1-phényl-5-(4-méthylsulfonylphényl)-3-trifluorométhyl-1H-pyrazole
[67] 4,5-dihydro-1-(2-fluorophényl)-5-(4-méthylsulfonylphényl)-3-trifluorométhyl-1H-pyrazole
[68] 1-(4-chloro-2-méthylphényl)-4,5-dihydro-5-(4-méthylsulfonylphényl)-3-trifluorométhyl-1H-pyrazole
[69] 4,5-dihydro-1-(3-fluorophényl)-5-(4-méthylsulfonylphényl)-3-trifluorométhyl-1H-pyrazole
[70] 4,5-dihydro-1-(3-méthylphényl)-5-(4-méthylsulfonylphényl)-3-trifluorométhyl-1H-pyrazole
[71] 4,5-dihydro-1-(2,4-diméthylphényl)-5-(4-méthylsulfonylphényl)-3-trifluorométhyl-1H-pyrazole
[72] 1-(2-chlorophényl)-4,5-dihydro-5-(4-méthylsulfonylphényl)-3-trifluorométhyl-1H-pyrazole
[73] 4,5-dihydro-1-(2-méthylphényl)-5-(4-méthylsulfonylphényl)-3-trifluorométhyl-1H-pyrazole
[74] 1-(2,4-dichlorophényl)-4,5-dihydro-5-(4-méthylsulfonylphényl)-3-trifluorométhyl-1H-pyrazole
[75] 4,5-dihydro-5-(4-méthylsulfonylphényl)-3-trifluorométhyl-1-(2-trifluorométhylphényl)-1H-pyrazole
[76] 5-(4-aminosulfonylphényl)-4,5-dihydro-1-(4-fluorophényl)-3-trifluorométhyl-1H-pyrazole
[77] 4,5-dihydro-1-phényl-3-méthyl-5-(4-méthylsulfonylphényl)-1H-pyrazole
[78] 4,5-dihydro-1-(4-fluorophényl)-5-(4-méthylsulfonylphényl)-1H-pyrazole
[81] (+)-4,5-dihydro-1-(4-fluorophényl)-5-(4-méthylsulfonylphényl)-3-trifluorométhyl-1H-pyrazole
[82] (-)-4,5-dihydro-1-(4-fluorophényl)-5-(4-méthylsulfonylphényl)-3-trifluorométhyl-1H-pyrazole
[83] (+)-1-(2,4-difluorophényl)-4,5-dihydro-5-(4-méthylsulfonylphényl)-3-trifluorométhyl-1H-pyrazole
[84] (-)-1-(2,4-difluorophényl)-4,5-dihydro-5-(4-méthylsulfonylphényl)-3-trifluorométhyl-1H-pyrazole ;
ou de l'un de ses sels physiologiquement acceptables,
dans la préparation d'un médicament pour prévenir ou traiter des maladies de prolifération cellulaire, en particulier pour prévenir ou traiter des processus pré-néoplasiques ou néoplasiques, une angiogenèse tumorale, une cachexie et des processus liés au facteur nécrosant les tumeurs (TNF) et, en général, des processus qui peuvent tirer avantage d'une inhibition de l'expression du gène responsable de la synthèse de la cyclooxygénase-2 (COX-2) chez les mammifères, y compris l'homme.

3. Utilisation selon la revendication 1 d'un composé de formule générale (I) ou de l'un de ses sels physiologiquement acceptables, conjointement avec un autre produit couramment utilisé dans le traitement de néoplasies, produisant dans ce cas une synergie, dans la préparation d'un médicament pour prévenir ou traiter des maladies de prolifération cellulaire, en particulier pour prévenir ou traiter des processus pré-néoplasiques ou néoplasiques, une angiogenèse tumorale, une cachexie et des processus liés au facteur nécrosant les tumeurs (TNF) et, en général, des processus qui peuvent tirer avantage d'une inhibition de l'expression du gène responsable de la synthèse de la cyclooxygénase-2 (COX-2) chez les mammifères, y compris l'homme.
